# EUROPEAN PATENT APPLICATION

(11) **EP 4 806 444 A1**
(43) Date of publication of application: **16.09.2026**
(21) Application number: 24888759.8
(22) Date of filing: 07.11.2024
(51) Int. Cl.: A61K 33/26, A61K 33/40, A61K 39/395, A61K 47/36, A61K 47/68, A61K 49/00, A61K 49/16, A61K 49/18, A61P 35/00

(54) **COMPLEX FOR TUMOR CHEMODYNAMIC THERAPY**

(30) Priority: 08.11.2023 JP 2023190866
(71) Applicant: NATIONAL UNIVERSITY CORPORATION TOKAI NATIONAL HIGHER EDUCATION AND RESEARCH SYSTEM, Nagoya-shi, Aichi 464-8601 (JP); JSR Corporation, Tokyo 105-8640 (JP)
(72) Inventor: SATO, Kazuhide, Nagoya-shi, Aichi 464-8601 (JP); SHIMADA, Mibuko, Tokyo 105-8640 (JP)
(74) Representative: TBK
(86) International application number: PCT/JP2024/039549
(87) International publication number: WO 2025/100466

(57) **Abstract**

A complex is used for tumor chemodynamic therapy, and the complex contains a nanoparticle that encloses iron oxide and has a hydrophilic polymer in a surface layer thereof; and target recognition molecule bonded to the nanoparticle, and is capable of binding to a target molecule of a tumor cell.

## Description

### Technical Field

The present invention relates to a complex for tumor chemodynamic therapy.

### Background Art

Chemodynamic therapy (CDT) is a therapeutic method of inducing cell death by generating reactive oxygen species (ROS) from endogenous H₂O₂ in a tumor microenvironment (TME) by a continuous chemical process to damage tumor cells. ROS generation by CDT is primarily based on Fenton reaction, and mainly Fe²⁺-based nanomaterials are used for CDT to promote the reaction. Transition metal-based nanomaterials such as Cu²⁺ have also been developed for CDT based on Fenton-like reactions. However, since TME is weakly acidic, it is an unsuitable condition for Fe²⁺-based Fenton reaction requiring strong acidity (pH of 2 to 4), and since the production of hydrogen peroxide (H₂O₂) is lower than normal cells, the therapeutic efficiency of conventional CDT is not necessarily sufficient.

Patent Literature 1 describes the use of polydopamine iron nanoparticles filled with artesunate coated with fibronectin for CDT, in which fibronectin enhances biocompatibility and targeting of nanomaterials, and chemotherapeutic agent artesunate promotes tumor apoptosis and promotes ROS generation by reacting with ferrous ions.

Patent Literature 2 describes that a magnetic nanoenzyme (Fc-MBL-rGO-Fe₃O₄) obtained by modifying the surface of a reduced graphene oxide-supported triiron tetraoxide magnetic nanoenzyme (rGO-Fe₃O₄) with mannose lectin (Fc-MBL) exhibits an antibacterial effect by using CDT and photothermal therapy (PTT) in combination.

Non-Patent Literature 1 describes that CuS nanoparticles have a PTT effect due to near-infrared light absorption and a CDT effect due to ROS production, the PTT effect due to CuS promotes ROS production thereof, and Gd₂O₃/CuS nanoparticles in which Gd₂O₃ for magnetic imaging and a fluorophore Cy5.5 for fluorescence imaging are modified, and selective recognition ability for tumor cells is imparted to CuS nanoparticles by bonding of RGD peptides.

Near-infrared photoimmunotherapy (NIR-PIT) is a treatment method in which an antibody complex in which a photosensitive substance reactive to near-infrared rays is introduced into an antibody specific to an antigen on the surface of a tumor cell is prepared, the antibody complex is bound to the tumor cell, and the tumor cell is selectively killed by locally irradiating the tumor cell with near-infrared rays. As the photosensitive substance, a chemical species containing a phthalocyanine skeleton (for example, so-called IR700 molecules such as IRDye700DX) is mainly used (see Patent Literatures 3 and 4 and Non-Patent Literature 2). The mechanism of NIR-PIT is different from cell death induction by ROS generated by light irradiation as in conventional light therapy, because cell death occurs in an extremely short time (2 to 6 minutes), and no damage is observed in mitochondria. In recent years, it has been clarified that the mechanism of the tumor therapeutic effect by NIR-PIT is as follows: the antibody complex bonded to the tumor cell surface aggregates by light irradiation, thereby disrupting cell membranes of the tumor cells, and as a result, a difference in osmotic pressure between the inside and outside of the cells occurs to induce cell death (Non-Patent Literature 2). Patent Literature 5 describes that a complex containing an antibody molecule to which a photosensitive substance containing a phthalocyanine skeleton is bonded, and magnetic particles or semiconductor particles bonded to the antibody molecule can be used for NIR-PIT and tumor imaging.

### Citation List

### Patent Literatures

Patent Literature 1: CN 115089560 A
Patent Literature 2: CN 115944727 A
Patent Literature 3: JP 2014-523907 A
Patent Literature 4: JP 2019-218374 A
Patent Literature 5: WO 2022/054798 A1

### Non-Patent Literatures

Non-Patent Literature 1: ACS Applied Materials & Interfaces, 2022, 14:34365-34376
Non-Patent Literature 2: 40th Japan Society for Laser Surgery and Medicine Awarded Article, "A Mechanism of Cancer Cell Cytotoxicity of Near-Infrared Photoimmunotherapy", The Journal of Japan Society for Laser Surgery and Medicine, 2020 Vol. 41, Issue 2, p. 104-109

### Summary of Invention

### Technical Problem

The present invention provides a nanoparticle complex for tumor chemodynamic therapy (CDT) and a method for treating a tumor by CDT using the complex.

### Solution to Problem

The present inventors have found that a nanoparticle complex in which target recognition molecule is conjugated to a nanoparticle enclosing iron oxide exerts a tumor therapeutic effect by CDT with light irradiation.

Therefore, the present invention includes the following embodiments.
[1] A complex for use in chemodynamic therapy of a tumor, the complex containing:
   a nanoparticle that encloses iron oxide and has a hydrophilic polymer in a surface layer thereof; and
   target recognition molecule(s) bonded to the nanoparticle, and is capable of binding to a target molecule of a tumor cell.
[2] The complex according to [1], in which the chemodynamic therapy is based on a photo-Fenton reaction generated by near-infrared light irradiation.
[3] The complex according to [1] or [2], which is further used for photothermal therapy.
[4] The complex according to any one of [1] to [3], which is further used for tumor imaging.
[5] The complex according to any one of [1] to [4], further having photosensitive site(s) bonded to the nanoparticle or the target recognition molecule, in which the photosensitive site contains a photosensitive group having a maximum absorption wavelength at 500 to 1500 nm, and one or more hydrophilic functional groups linked to or coordinated with the photosensitive group.
[6] The complex according to [5], in which the photosensitive site is bonded to the target recognition molecule.
[7] The complex according to [5] or [6], which is further used for photoimmunotherapy.
[8] The complex according to any one of [1] to [7], in which the target recognition molecule is an antibody.
[9] The complex according to any one of [1] to [8], in which the hydrophilic polymer is a polysaccharide.
[10] The complex according to [9], in which the polysaccharide is dextran.
[11] The complex according to any one of [1] to [10], in which a content of iron atoms in the complex is 30 mass% or more.
[12] The complex according to any one of [1] to [11], in which the number of bonds of the target recognition molecule to the nanoparticle is 1 to 20 per nanoparticle.
[13] The complex according to any one of [1] to [12], in which the target molecule of the tumor cell is an epidermal growth factor receptor, an EGF receptor family, or a platelet-activating receptor.
[14] A composition for use in chemodynamic therapy of a tumor, the composition containing the complex according to any one of [1] to [13].
[15] The composition according to [14], further containing hydrogen peroxide.
[16] The composition according to [14] or [15], further containing a biological reducing substance.
[17] The composition according to any one of [14] to [16], which is further used for photothermal therapy.
[18] The composition according to any one of [14] to [17], which is further used for tumor imaging.
[19] The composition according to any one of [14] to [18], in which the complex has photosensitive site(s), and the photosensitive site contains a photosensitive group having a maximum absorption wavelength at 500 to 1500 nm, and one or more hydrophilic functional groups linked to or coordinated with the photosensitive group.
[20] The composition according to [19], which is further used for photoimmunotherapy.
[21] Use of the complex according to any one of [1] to [13] in production of a tumor therapeutic agent for tumor chemodynamic therapy.
[22] The use according to [21], in which the tumor therapeutic agent is further used for photothermal therapy.
[23] The use according to [21] or [22], in which the tumor therapeutic agent is further used for tumor imaging.
[24] The use according to any one of [21] to [23], in which the complex has photosensitive site(s), and the photosensitive site contains a photosensitive group having a maximum absorption wavelength at 500 to 1500 nm, and one or more hydrophilic functional groups linked to or coordinated with the photosensitive group.
[25] The use according to [24], in which the tumor therapeutic agent is further used for photoimmunotherapy.
[26] The use according to any one of [21] to [25], in which a composition containing the complex and hydrogen peroxide is used.
[27] The use according to any one of [21] to [26], in which a composition containing the complex and a biological reducing substance is used.
[28] A method for treating a tumor by chemodynamic therapy, the method including:
   a step of administering the complex according to any one of [1] to [13] to a patient in need of tumor chemodynamic therapy; and
   a step of irradiating the patient with light having a wavelength of 500 to 1500 nm.
[29] The method according to [28], wherein hydrogen peroxide is further administered to the patient together with the complex.
[30] The method according to [28] or [29], in which a biological reducing substance is further administered to the patient together with the complex.

### Advantageous Effects of Invention

The complex of the present invention exhibits a tumor therapeutic effect by CDT with light irradiation.

When the complex of the present invention further has photosensitive site, since the complex exerts a tumor therapeutic effect by photothermal therapy (PTT) or near-infrared photoimmunotherapy (NIR-PIT) according to the light irradiation, a combined effect by these therapeutic methods can be enjoyed by using the complex. Since the complex of the present invention can be used for tumor imaging, it enables observation of a target tumor in a patient's body or optimization of light irradiation for CDT.

### Brief Description of Drawings

Fig. 1 shows zeta potentials of Nanomag-D-spio and Nanomag-D-spio-pan-IR700.
Fig. 2 shows an image (top) observed with an atomic force microscope (AFM) of Nanomag-D-spio and Nanomag-D-spio-pan-IR700, a measured size (bottom), and an estimated shape (middle drawing).
Fig. 3 shows magnetic susceptibility of Nanomag-D-spio and Nanomag-D-spio-pan-IR700 by VSM measurement.

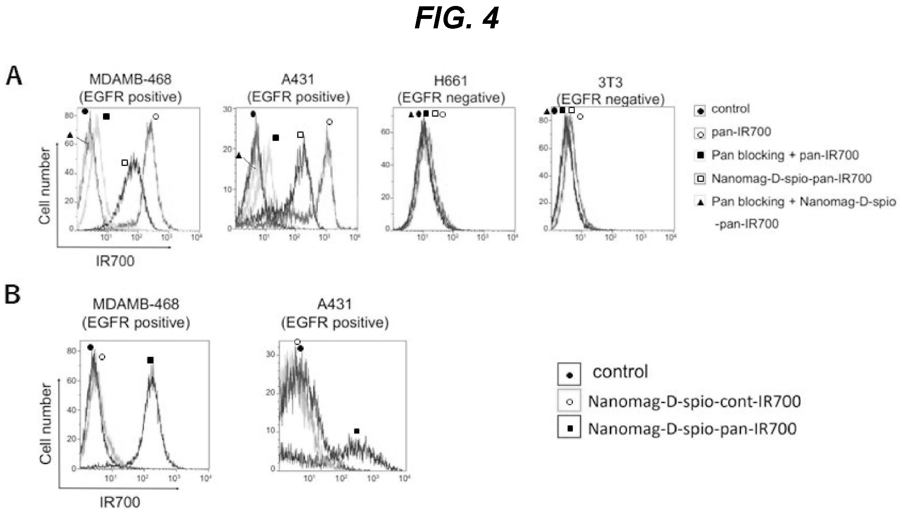

Fig. 4 shows specific binding of Nanomag-D-Spio-pan-IR700 to EGFR. A: The number of IR700-labeled cells in EGFR-expressing (EGFR positive) cells (MDAMB468 and A431) and EGFR-non-expressing (EGFR negative) cells (H661 and 3T3). pan-IR700; pan-IR700 added cells. Nanomag-D-Spio-pan-IR700; Nanomag-D-Spio-pan-IR700 added cells. Pan blocking; cells to which pan-IR700 or Nanomag-D-Spio-pan-IR700 was added in the presence of an EGFR inhibitor. B: The number of IR700-labeled cells in EGFR-expressing (EGFR positive) cells (MDAMB468 and A431) in which Nanomag-D-Spio-pan-IR700 or Nanomag-D-Spio-cont-IR700 (cont; a ligand (IgG antibody) other than an EGFR ligand) was added.
Fig. 5 shows a fluorescence intensity of Nanomag-D-Spio-pan-IR700. A: in SDS, and B: in serum. T-test was performed (*; p < 0.001, ns; no significant difference, vs control, n = 3).
Fig. 6 shows fluorescence images of Nanomag-D-Spio-pan-IR700 added cells. Hoechst; Hoechst staining, Lyso tracker; lysosome staining, IR700; IR700-labeled, Merge; superimposed image. DIC; bright-field image by optical microscope. White bar; 30 µm.
Fig. 7 shows an absorbance of a Nanomag-D-Spio-containing methylene blue solution. A: change in absorbance by laser irradiation. B: change in absorbance with heat treatment time in the presence of reduced glutathione.
Fig. 8 shows an exothermic behavior of a complex with respect to laser irradiation. A: temperature rise curve of a complex-containing liquid, error bar; SEM, T-test (*; p < 0.05, vs Pan-IR700, n = 3) was performed. B: left; infrared thermographic image of Nanomag-D-Spio-pan-IR700-containing liquid after laser irradiation, right; liquid temperature of Nanomag-D-Spio-pan-IR700-containing liquid after laser irradiation.
Fig. 9 shows fluorescence images of in vitro disease model cells (A431, MDAMB468, and PC-9) administered with Nanomag-D-Spio-pan-IR700. Hoechst; Hoechst staining, PI; pyridinium iodide staining, IR700; IR700-labeled, Merge; superimposed image. DIC; bright-field image by optical microscope. White bar; 30 µm.
Fig. 10A shows fluorescence images of disease model cells contained in in vitro spheroids administered with Nanomag-D-Spio-pan-IR700. Spheroids were prepared by mixing disease model cells and 3T3-RFP at 1/1 (volume ratio). A431 was used as a disease model cell. 3T3-RFP was prepared by transfecting 3T3 with the gene for red fluorescent protein (RFP). RFP; RFP-labeled, IR700; IR700-labeled, SYTOX Blue; SYTOX^{™} Blue staining, Merge; superimposed image. DIC; bright-field image by optical microscope. White bar; 30 µm.
Fig. 10B is the same as Fig. 10A. MDAMB468 or PC-9 was used as a disease model cell.
Fig. 11A shows a protocol for Experiment 10. Top: schedule of complex administration to A431-transplanted xenograft model mice, laser irradiation, and tumor assessment by luciferase assay (Bioluminescent Imaging; BLI). Bottom: illustration of the process of A431 transplantation into mice, complex administration, and laser irradiation.
Fig. 11B shows images of complex-administered xenograft model mice obtained by luciferase assay observation.
Fig. 11C shows a change in tumor size in complex-administered xenograft model mice evaluated by luciferase assay. RLU in a region of interest of the image of the tumor on Day 0 is taken as 100, and the change in tumor size is expressed as an RLU ratio. Error bar; SEM.
Fig. 11D shows a change in tumor size in complex-administered xenograft model mice. The tumor size was measured after removing tumors from mice. Error bar; SEM.
Fig. 12A shows a protocol for Experiment 11. Left: illustration of the process of spheroid transplantation into mice and laser irradiation. Right: schedule of complex administration to spheroid-transplanted xenograft model mice, laser irradiation, and tumor assessment by luciferase assay (BLI).
Fig. 12B shows images of complex-administered xenograft model mice obtained by luciferase assay observation.
Fig. 12C shows a change in tumor size in complex-administered xenograft model mice evaluated by luciferase assay. RLU in a region of interest of the image of the tumor on Day 0 is taken as 100, and the change in tumor size is expressed as an RLU ratio. Error bar; SEM.
Fig. 12D shows a change in tumor size in complex-administered xenograft model mice. The tumor size is measured after removing the tumors from mice, and a relative value is represented with the tumor size on Day 0 as 100. Error bar; SEM.
Fig. 13 shows a nuclear magnetic resonance intensity of an MRI image. Dunnett's test was performed using control as a control group (*p < 0.0001, n = 5).
Fig. 14 shows A: change in absorbance of a Nanomag-D-Spio-Pan-containing methylene blue solution by laser irradiation, and B: change in absorbance of a Nanomag-D-Spio-Tra-containing methylene blue solution by laser irradiation.

### Description of Embodiments

All patent literatures, non-patent literatures, and other publications cited herein are incorporated herein by reference in their entirety.

In the present specification, the term "tumor" refers to tumors in general including benign and malignant tumors, and epithelial and nonepithelial tumors, and sites of occurrence thereof (tissues and organs) are also not particularly limited. A tumor to be targeted in the present invention is preferably a malignant tumor such as cancer. The cancer may be a liquid tumor or a solid tumor, and may include all kinds of cancers, such as epithelial cancer, adenocarcinoma, sarcoma, and malignant lymphoma. Examples of the tumor include liquid tumors, including acute leukemia (such as acute lymphocytic leukemia, acute myelogenous leukemia, myeloblastic leukemia, promyelocytic leukemia, myelomonocytic leukemia, monocytic leukemia, and erythroleukemia), chronic leukemia (such as chronic myelogenous (granulocytic) leukemia, chronic lymphocytic leukemia, and hairy cell leukemia), T-cell prolymphocytic leukemia, large granular lymphocytic leukemia, adult T-cell leukemia, polycythemia vera, Hodgkin lymphoma, non-Hodgkin lymphoma, multiple myeloma, Waldenstrom macroglobulinemia, heavy chain disease, and the like; and solid tumors, including fibrosarcoma, myxosarcoma, liposarcoma, chondrosarcoma, osteogenic sarcoma, other sarcomas, synovioma, mesothelioma, Ewing's tumor, leiomyosarcoma, rhabdomyosarcoma, colon cancer, pancreatic cancer, breast cancer, ovarian cancer, prostate cancer, hepatocellular carcinoma, lung cancer, colorectal cancer, squamous cell carcinoma, basal cell carcinoma, adenocarcinoma (e.g., adenocarcinoma of the pancreas, colon, ovary, lung, breast, stomach, prostate, uterine cervix, or esophagus), sweat gland cancer, sebaceous adenocarcinoma, papillary cancer, papillary adenocarcinoma, medullary cancer, bronchogenic cancer, renal cell carcinoma, hepatoma, bile duct cancer, choriocarcinoma, Wilms tumor, cervical cancer, testicular tumor, bladder cancer, glioma, astrocytoma, medulloblastoma, craniopharyngioma, ependymoma, pinealoma, hemangioblastoma, acoustic neurinoma, oligodendroglioma, meningioma, melanoma, neuroblastoma, retinoblastoma, and the like. The tumor in the present specification may be a primary tumor or a recurrent tumor.

In the present specification, the term "tumor-affected area" or "affected area" refers to a tumor tissue in which tumor cells are mainly present. The tumor tissue includes a tissue formed of tumor cells and a tissue in which tumor cells are mixed with normal cells or a normal tissue. When the tumor tissue has normal cells or a normal tissue mixed therein, the ratio of the volume or cell count of the tumor cells to the normal cells or the normal tissue is not particularly limited.

In the present specification, the term "antibody" refers to a polypeptide ligand including at least one light chain variable region and/or heavy chain variable region that specifically recognizes an epitope of an antigen and binds thereto. For example, in the present specification, the term "antibody" includes immunoglobulins of any classes, such as IgG, IgA, IgD, IgE, IgM, and subclasses thereof, and variants thereof, and further includes a chimeric antibody such as a humanized antibody, any other immunoglobulin modification product containing an antigen recognition site, and the like. In the present specification, the term "antibody" contains a fragment or domain of an immunoglobulin containing an antigen recognition site, such as a Fab fragment, a Fab' fragment, an F(ab)'2 fragment, a single-chain Fv ("scFv"), a disulfide-stabilized Fv ("dsFv"), a VHH (variable domain of heavy chain of heavy chain antibody), or VNAR (single variable new antigen receptor domain antibody).

The "chemodynamic therapy" (CDT) in the present specification is a therapeutic method of inducing cell death by generating reactive oxygen species (ROS), such as superoxide (·O), singlet oxygen (O), and hydroxy radical (·OH), from H₂O₂ in a tumor microenvironment (TME) by a continuous chemical process to damage tumor cells by oxidative stress. It is presumed that ROS generation by CDT is basically based on the Fenton reaction.

The "photothermal therapy" (PTT) in the present specification is a therapeutic method in which cells to which a photothermal material that generates heat by light irradiation is administered are irradiated with light, and the cells are killed by heat released from the photothermal material. The therapeutic effect by PTT depends on the difference between the upper limit of the survival temperature of cancer cells (about 42°C) and the upper limit of normal cells (45°C).

The "near-infrared photoimmunotherapy" (NIR-PIT) in the present specification is a therapeutic method in which an antibody complex in which a photosensitive substance that reacts with near infrared rays is introduced into an antibody specific to an antigen on the surface of a tumor cell is bound to the tumor cell and irradiated with near infrared rays to induce cell death by a light response of the photosensitive substance. In recent years, it has been clarified that the mechanism of the tumor therapeutic effect of NIR-PIT is as follows: the photosensitive substance contained in the antibody complex bonded to the tumor cell surface reacts with near infrared rays to cause aggregation of the antibody complex, thereby disrupting cell membranes of the tumor cells, and as a result, a difference in osmotic pressure between the inside and outside of the cells occurs to induce cell death (Non-Patent Literature 2).

### 1. Complex

In one embodiment, the present invention provides a complex used as an agent for tumor chemodynamic therapy (CDT). The complex provided by the present invention contains a nanoparticle, and target recognition molecule bonded to the nanoparticle, and is capable of binding to a target molecule of a tumor cell.

### 1.1. Nanoparticle

The nanoparticle used in the complex of the present invention encloses iron oxide and has a hydrophilic polymer in a surface layer thereof. Since the nanoparticle has a hydrophilic polymer in the surface layer, a hydration layer is formed around the hydrophilic polymer. The hydration layer suppresses non-specific adsorption of protein to the nanoparticle, thereby avoiding capture and decomposition of the nanoparticle by phagocytic cells (imparting stealth), and thus improving blood retention of the nanoparticle. Since the nanoparticle forms a hydration layer, the incorporation of the complex of the present invention into cells by endocytosis is promoted. Examples of the iron oxide enclosed in the nanoparticle include FeO, Fe₂O₃, and Fe₃O₄. Fe2_{O}3 or Fe₃O₄ is preferable. Examples of the hydrophilic polymer include polyacrylamide, poly(vinyl alcohol), polyethylene glycol, agarose, polysaccharides, glycoproteins, and proteins such as heparin and albumin, and polysaccharide is preferable since it can permeate a water-soluble substance such as hydrogen peroxide or a biological reducing substance, and ions such as Fe²⁺. Examples of the polysaccharides include dextran, hyaluronic acid, alginic acid, and pullulan, and dextran is preferable. The form of the hydrophilic polymer in the surface layer includes a gelled form.

The nanoparticle may have, on the surface layer thereof, a reactive group or linker that is responsible for bonding to target recognition molecule described later. For example, the nanoparticle preferably has, on the surface layer thereof, a reactive functional group such as an amino group or a carboxy group, or a protein-binding molecule such as avidin, streptavidin, or protein A.

The nanoparticle is preferably a particle having an average particle size of 500 nm or less.

The "average particle size" of the particle in the present specification is an average particle size measured by a dynamic light scattering method. The average particle size of the nanoparticles can be appropriately adjusted depending on the type of cancer in view of, for example, an enhanced permeation and retention effect (EPR effect). For example, the average particle size of the nanoparticles is preferably 200 nm or less, more preferably 100 nm or less, and still more preferably 50 nm or less. The lower limit of the average particle size of the nanoparticles is not particularly limited, but is preferably 1 nm or more, more preferably 10 nm or more, and still more preferably 20 nm or more from the viewpoint of manufacturability.

The content of the iron oxide contained in the nanoparticle used in the complex of the present invention is preferably 30 mass% or more, and more preferably 35 mass% or more, and is preferably 80 mass% or less, and more preferably 70 mass% or less in terms of the content of iron atoms in the total mass of the complex of the present invention. When the content of iron atoms in the complex is too large, the water solubility or dispersibility of the complex tends to decrease. On the other hand, when the content of iron atoms in the complex is too small, the therapeutic effect by CDT tends to decrease.

As the nanoparticle used in the complex of the present invention, a commercially available product can be purchased. For example, dispersions of dextran coated superparamagnetic iron oxide particles are sold by micromod Partikeltechnologie GmbH as Nanomag-D-spio series.

### 1.2. Target recognition molecule

The target recognition molecule contained in the complex of the present invention is a molecule for binding the complex to a tumor cell which is a therapeutic target. The target recognition molecule recognizes and binds to a target molecule (receptor) present in a tumor cell. Examples of the target recognition molecule include a low-molecular-weight compound, a peptide, an antibody, a ligand such as an antigen-binding fragment, an aptamer, a sugar chain, and podoplanin. The target recognition molecule is preferably an antibody. The type of the antibody can be appropriately selected according to an antigen (target molecule) present on the surface of a tumor cell.

Preferred examples of the antigen include a cell transmembrane protein present in tumor cells. Examples of the cell transmembrane protein include tumor-specific proteins (also known as tumor-specific antigens in the art) expressed on the surface of tumor cells. The tumor-specific proteins are proteins that are unique to cancer cells or more abundant in cancer cells as compared to other cells such as normal cells.

Examples of the tumor-specific proteins include a member of an epidermal growth factor receptor (EGFR) family (for example, HER1, 2, 3, and 4), and a member of a cytokine receptor (such as CD20, CD25, IL-13R, CD5, or CD52).

Specific examples of the tumor-specific proteins include human epidermal growth factor receptor 2 (HER-2, for example, GenBank Accession Nos. M16789.1, M16790.1, M16791.1, M16792.1, and AAA58637), which is associated with breast cancer, ovarian cancer, stomach cancer, and uterine cancer; and HER-1 (for example, GenBank Accession Nos. NM_005228 and NP_005219), which is associated with lung cancer, anal cancer, and glioma as well as adenocarcinoma.

Other specific examples of the tumor-specific proteins include CD52 (for example, GenBank Accession Nos. AAH27495.1 and CAI15846.1), which is associated with chronic lymphocytic leukemia; CD33 (for example, GenBank Accession Nos. NM_023068 and CAD36509.1), which is associated with acute myelogenous leukemia; and CD20 (for example, GenBank Accession Nos. NP_068769 and NP_031667), which is associated with non-Hodgkin lymphoma.

Other specific examples of the tumor-specific proteins include any of various MAGEs (melanoma-associated antigen E) including MAGE1 (for example, GenBank Accession Nos. M77481 and AAA03229), MAGE2 (for example, GenBank Accession Nos. L18920 and AAA17729), MAGE3 (for example, GenBank Accession Nos. U03735 and AAA17446), MAGE4 (for example, GenBank Accession Nos. D32075 and A06841.1), and the like; any of various tyrosinases (for example, GenBank Accession Nos. U01873 and AAB60319); mutant ras; mutant p53 (for example, GenBank Accession Nos. X54156, CAA38095, and AA494311); p97 melanoma antigen (for example, GenBank Accession Nos. M12154 and AAA59992); human milk fat globule (HMFG) (for example, GenBank Accession Nos. S56151 and AAB19771), which is associated with breast tumor; any of various BAGEs (human B-melanoma-associated antigen E) including BAGE1 (for example, GenBank Accession No. Q13072) and BAGE2 (for example, GenBank Accession Nos. NM_182482 and NP_872288); gp100 (for example, GenBank Accession Nos. S73003 and AAC60634), which is associated with melanoma; MART1 antigen (for example, GenBank Accession No. NP_005502), which is associated with melanoma; any of various GAGEs (G antigen) including GAGE1 (for example, GenBank Accession No. Q13065), or any of GAGE2 to GAGE6; various gangliosides; and CD25 (for example, GenBank Accession Nos. NP_000408.1 and NM_000417.2).

Other specific examples of the tumor-specific proteins include HPV16/18 and E6/E7 antigens (for example, GenBank Accession Nos. NC_001526, FJ952142.1, ADB94605, ADB94606, and U89349), which are associated with cervical cancer; mucin (MUC1)-KLH antigen (for example, GenBank Accession Nos. J03651 and AAA35756), which is associated with breast cancer; carcinoembryonic antigen (CEA) (for example, GenBank Accession Nos. X98311 and CAA66955), which is associated with colorectal cancer; cancer antigen 125 (CA125, or also known as mucin 16 or MUC16) (for example, GenBank Accession Nos. NM_024690 and NP_078966), which is associated with ovarian cancer and any other cancer; alpha-fetoprotein (AFP) (for example, GenBank Accession Nos. NM_001134 and NP_001125), which is associated with liver cancer; Lewis Y antigen, which is associated with colorectal cancer, biliary tract cancer, breast cancer, small cell lung cancer, and any other cancer; tumor-associated glycoprotein 72(TAG72), which is associated with adenocarcinoma; and PSA antigen (for example, GenBank Accession Nos. X14810 and CAA32915), which is associated with prostate cancer.

Other specific examples of the tumor-specific proteins include prostate membrane specific antigen (PMSA; for example, GenBank Accession Nos. AAA60209 and AAB81971.1), which is associated with prostate cancer; NY-ESO-1 (for example, GenBank Accession Nos. U87459 and AAB49693), which is associated with melanoma, sarcoma, testicular cancer, and any other cancer; hTERT (alias: telomerase) (for example, GenBank Accession Nos. NM_198253 and NP_937983 (variant 1), NM_198255 and NP_937986 (variant 2)); proteinase 3 (for example, GenBank Accession Nos. M29142, M75154, M96839, X55668, NM00277, M96628, X56606, CAA39943, and AAA36342); and Wilms' tumor 1 (WT-1, for example, GenBank Accession Nos. NM_000378 and NP_000369 (variant A), NM_024424 and NP_077742 (variant B), NM_024425 and NP_077743 (variant C), and NM_024426 and NP_077744 (variant D)).

Other specific examples of the tumor-specific proteins include PD-L1 and PD-L2, which are associated with immune checkpoint.

The tumor-specific proteins described in the present specification are named following the GenBank database of National Center for Biotechnology Information (NCBI)
([www.ncbi.nlm.nih.gov/genbank/]).

Examples of the antibody that is the target recognition molecule which may be contained in the complex of the present invention include, but are not limited to, cetuximab, panitumumab, zalutumumab, nimotuzumab, trastuzumab, Ado-trastuzumab emtansine, tositumomab, rituximab, ibritumomab tiuxetan, daclizumab, gemtuzumab, alemtuzumab, a CEA-scan Fab fragment, an OC125 monoclonal antibody, ab75705, B72.3, bevacizumab, afatinib, axitinib, bosutinib, cabozantinib, ceritinib, crizotinib, dabrafenib, dasatinib, erlotinib, everolimus, ibrutinib, imatinib, lapatinib, lenvatinib, nilotinib, olaparib, palbociclib, pazopanib, pertuzumab, ramucirumab, regorafenib, ruxolitinib, sorafenib, sunitinib, temsirolimus, trametinib, vandetanib, vemurafenib, vismodegib, basiliximab, ipilimumab, nivolumab, pembrolizumab, MPDL3280A, pidilizumab (CT-011), MK-3475, BMS-936559, MPDL3280A (atezolizumab), tremelimumab, IMP321, BMS-986016, LAG525, urelumab, PF-05082566, TRX518, MK-4166, dacetuzumab (SGN-40), lucatumumab (HCD122), SEA-CD40, CP-870, CP-893, MEDI6469, MEDI6383, MEDI4736, MOXR0916, AMP-224, PDR001, avelumab (MSB0010718C), rHIgM12B7, ulocuplumab, BKT140, varlilumab (CDX-1127), ARGX-110, MGA271, lirilumab (BMS-986015, IPH2101), IPH2201, AGX-115, emactuzumab, CC-90002, and MNRP1685A, and fragments including antigen recognition sites thereof.

In the complex of the present invention, the number of bonds of the target recognition molecule to the nanoparticle is preferably 1 or more, and is preferably 20 or less, more preferably 16 or less, still more preferably 12 or less, and even more preferably 5 or less per particle. When the number of bonds of the target recognition molecule to the nanoparticle is too large, the size of the complex as a whole is increased, and thus it tends to be difficult for the complex to be selectively distributed in tumor cells. The lower limit of the number of bonds of the target recognition molecule in 1 mg of the complex of the present invention is preferably 1.0×10⁻¹² mol/mg, more preferably 5.0×10⁻¹² mol/mg, and still more preferably 1.0×10⁻¹¹ mol/mg. Meanwhile, the upper limit of the number of bonds of the target recognition molecule in 1 mg of the complex of the present invention is preferably 1.0×10⁻⁸ mol/mg, more preferably 5.0×10⁻⁹ mol/mg, and still more preferably 1.0×10⁻⁹ mol/mg.

### 1.3. Photosensitive site

The complex of the present invention can further have photosensitive site. The photosensitive site is bonded to the nanoparticle or the target recognition molecule in the complex of the present invention. The photosensitive site contains a photosensitive group having a maximum absorption wavelength at 500 to 1500 nm, and one or more hydrophilic functional groups linked to or coordinated with the photosensitive group. It is presumed that the hydrophobicity of the photosensitive site is increased by irradiation with a near-infrared ray (NIR), for example, a light beam having a wavelength of 500 to 1500 nm. More specifically, it is presumed that when light having such wavelength is radiated, the photochemical reaction of the photosensitive group dissociates the hydrophilic functional group or changes the structure, and the hydrophobicity of the photosensitive site increases.

Examples of the hydrophilic functional group contained in the photosensitive site include, but are not limited to, a carboxylate (-CO₂⁻) group, a sulfonate (-SO₃⁻) group, a sulfonyl (-SO₂⁻) group, a sulfate (-SO4⁻²) group, a hydroxy (-OH) group, a phosphate (-OPO₃⁻²) group, a phosphonate (-PO₃⁻²) group, an amino (-NH₂) group, and substituted or unsubstituted quaternary nitrogen (each having any counterion). Examples of the counterion include, but are not limited to, sodium, potassium, calcium, ammonium, an organic amino acid, and magnesium. The photosensitive site may further have a reactive group or linker for bonding to the target recognition molecule or nanoparticle.

Examples of the photosensitive site used in the complex of the present invention include a site containing a phthalocyanine skeleton. Phthalocyanine is azaporphyrin (that is, C₃₂H₁₆N₈) containing four benzoindole groups connected by nitrogen bridges in a 16-membered ring in which carbon atoms and nitrogen atoms are alternately arranged. Phthalocyanine forms a stable chelate with each of a metal cation and a non-metal cation, and in this case, the center of the ring is occupied by an element capable of holding one or two ligands. The periphery of the ring may be unsubstituted or substituted.

Preferably, a site containing a phthalocyanine skeleton used in the present invention is water-soluble and has at least one water-based solubilizing moiety. Preferably, the water-based solubilizing moiety of the site containing a phthalocyanine skeleton contains silicon. Preferably, the phthalocyanine skeleton has a core element such as Si, Ge, Sn, or Al at the center of the ring.

The site containing a phthalocyanine skeleton used in the present invention has a maximum absorption wavelength of preferably 500 to 1500 nm, more preferably 600 to 850 nm, and still more preferably 660 to 740 nm. The site containing a phthalocyanine skeleton preferably has one or more ligands containing a hydrophilic functional group. Examples of the hydrophilic functional group include, but are not limited to, a carboxylate (-CO₂⁻) group, a sulfonate (-SO₃⁻) group, a sulfonyl (-SO₂⁻) group, a sulfate (-SO₄⁻²) group, a hydroxy (-OH) group, a phosphate (-OPO₃⁻²) group, a phosphonate (-PO₃⁻²) group, an amino (-NH₂) group, and substituted or unsubstituted quaternary nitrogen (each having any counterion). Examples of the counterion include, but are not limited to, sodium, potassium, calcium, ammonium, an organic amino acid, and magnesium.

Preferably, the site containing a phthalocyanine skeleton used in the present invention contains a linker having a reactive group capable of forming a bond with the target recognition molecule or nanoparticle. That is, the site has a structure of a linker-phthalocyanine skeleton (L-D). Preferably, the site containing the phthalocyanine skeleton is bonded to the target recognition molecule or nanoparticle via the linker substituted on the periphery of the ring of the phthalocyanine skeleton.

In a preferred embodiment, the site containing a phthalocyanine skeleton used in the present invention is a compound represented by the following formula (Ia): 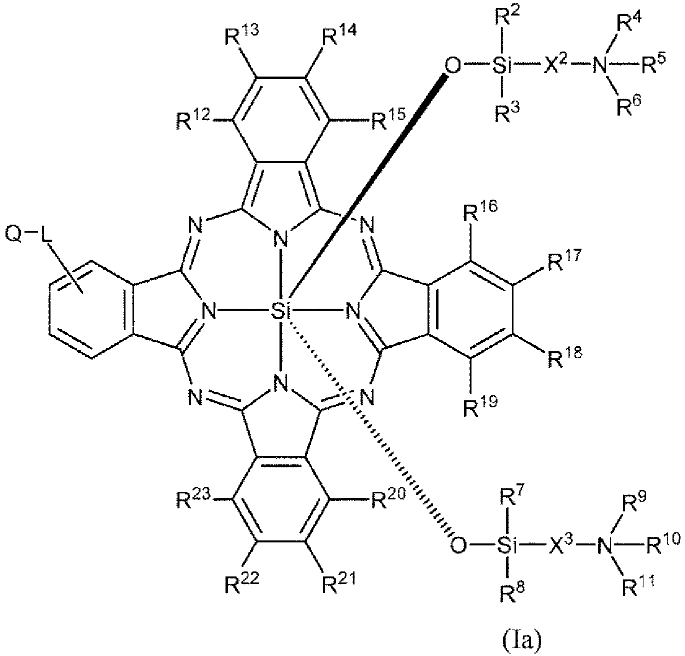 wherein,
L is a direct bond or a linker;
Q is a reactive group forming a bond with the target recognition molecule or nanoparticle;
R², R³, R⁷, and R⁸ are each independently selected from a substituted or unsubstituted alkyl and a substituted or unsubstituted aryl;
R⁴, R⁵, R⁶, R⁹, R¹⁰, and R¹¹, when present, are each independently selected from hydrogen, a substituted or unsubstituted alkyl, a substituted or unsubstituted alkanoyl, a substituted or unsubstituted alkoxycarbonyl, a substituted or unsubstituted alkylcarbamoyl, and a chelating ligand, where at least one of R⁴, R⁵, R⁶, R⁹, R¹⁰, and R¹¹ contains a water-soluble group;
R¹², R¹³, R¹⁴, R¹⁵, R¹⁶, R¹⁷, R¹⁸, R¹⁹, R²⁰, R²¹, R²², and R²³ are each independently selected from hydrogen, a halogen, a substituted or unsubstituted alkylthio, a substituted or unsubstituted alkylamino, and a substituted or unsubstituted alkoxy, or at least one of i) R¹³ and R¹⁴, and carbon atoms to which R¹³ and R¹⁴ are bonded, ii) R¹⁷ and R¹⁸, and carbon atoms to which R¹⁷ and R¹⁸ are bonded, and iii) R²¹ and R²², and carbon atoms to which R²¹ and R²² are bonded, forms a fused ring; and
X² and X³ are each independently a C₁ to C₁₀ alkylene with or without a heteroatom interposed between carbon-carbon bonds. Note that, in the present specification, the C₁ to C₁₀ alkylene means a methylene group and an alkylene group having 2 to 10 carbon atoms.

In one embodiment, L is a linker. In one embodiment, the linker is a linear or branched, cyclic or heterocyclic, saturated chain or unsaturated chain having 1 to 60 atoms, for example, 1 to 45 atoms or 1 to 25 atoms. In some cases, the atoms of the linker can each be selected from C, N, P, O, and S. In one embodiment, L can have additional hydrogen atoms satisfying a valence (in addition to the above-mentioned 1 to 60 atoms). In general, the linker may contain an ether, a thioether, an amine, an ester, a carbamate, urea, thiourea, carbonyl, an amide, a single bond, a double bond, a triple bond, an aromatic carbon-carbon bond, a phosphorus-oxygen bond, a phosphorus-sulfur bond, a nitrogen-nitrogen bond, a nitrogen-oxygen bond, a nitrogen-platinum bond, an aromatic bond, a heteroaromatic bond, or any combination thereof.

In one embodiment, L is represented by the formula -R¹-Y-X¹-Y¹-, wherein, R¹ is a divalent group or a direct bond; Y and Y¹ are each independently selected from a direct bond, oxygen, a substituted or unsubstituted nitrogen, and sulfur; and X¹ is selected from a direct bond and a C₁ to C₁₀ alkylene with or without a heteroatom interposed between the carbon-carbon bonds. Examples of the divalent group include, but are not limited to, a substituted or unsubstituted alkylene, a substituted or unsubstituted alkyleneoxycarbonyl, a substituted or unsubstituted alkylenecarbamoyl, a substituted or unsubstituted alkylenesulfonyl, and a substituted or unsubstituted arylene.

Specific examples of R¹ include, but are not limited to, a substituted or unsubstituted alkylene, a substituted or unsubstituted alkyleneoxycarbonyl, oxycarbonylamino, a substituted or unsubstituted alkylenecarbamoyl, a substituted or unsubstituted alkylenesulfonyl, a substituted or unsubstituted alkylenesulfonylcarbamoyl, a substituted or unsubstituted arylene, a substituted or unsubstituted arylenesulfonyl, a substituted or unsubstituted aryleneoxycarbonyl, a substituted or unsubstituted arylenecarbamoyl, a substituted or unsubstituted arylenesulfonylcarbamoyl, a substituted or unsubstituted carboxyalkyl, a substituted or unsubstituted carbamoyl, carbonyl, a substituted or unsubstituted heteroarylene, a substituted or unsubstituted heteroaryleneoxycarbonyl, a substituted or unsubstituted heteroarylenecarbamoyl, a substituted or unsubstituted heteroarylenesulfonylcarbamoyl, a substituted or unsubstituted sulfonylcarbamoyl, thiocarbonyl, sulfonyl, and sulfinyl.

An alkylene contained in each of the substituted or unsubstituted alkylene, the substituted or unsubstituted alkyleneoxycarbonyl, the substituted or unsubstituted alkylenecarbamoyl, the substituted or unsubstituted alkylenesulfonyl, and the substituted or unsubstituted alkylenesulfonylcarbamoyl is preferably a C₁ to C₁₀ alkylene with or without a heteroatom interposed between the carbon-carbon bonds.

In one embodiment, Q contains a reactive group for forming a bond with the target recognition molecule or nanoparticle. The term "reactive group" in the present specification refers to a moiety on a compound capable of forming a bond by chemically reacting with a functional group on a different material (for example, the target recognition molecule). The reactive group is typically an electrophile or nucleophile capable of forming a covalent bond through exposure to a corresponding functional group serving as a nucleophile or an electrophile, respectively.

In one embodiment, Q contains a reactive group that is reactive with a carboxyl group, an amino group, or a thiol group on the target recognition molecule or nanoparticle to which it binds. Suitable examples of the reactive group include, but are not limited to, an activated ester, a halogenated acyl, a halogenated alkyl, an anhydride, a carboxylic acid, a carbodiimide, a carbonate, a carbamate, a haloacetamide (for example, iodoacetamide), an isocyanate, an isothiocyanate, maleimide, an N-hydroxysuccinimide (NHS) ester, a phosphoramidite, a platinum complex, a sulfonic acid ester, and a thiocyanate. In one embodiment, the reactive group is a sulfhydryl-reactive chemical group, for example, maleimide, haloacetyl, or pyridyl disulfide. In one embodiment, the reactive group is amine-reactive. In a preferred embodiment, the reactive group is an NHS ester.

In one embodiment, R², R³, R⁷, and R⁸ are each independently a substituted or unsubstituted alkyl, for example, a substituted or unsubstituted methyl, ethyl, or isopropyl.

In one embodiment, at least one of R⁴, R⁵, R⁶, R⁹, R¹⁰, and R¹¹ contains a water-soluble group. In some cases, at least two of R⁴, R⁵, R⁶, R⁹, R¹⁰, and R¹¹ each contain a water-soluble group, and in other cases, three or more thereof each contain a water-soluble group. In one embodiment, at least one of R⁴, R⁵, R⁶, R⁹, R¹⁰, and R¹¹ is an alkyl substituted with a water-soluble group. In one embodiment, R⁴, R⁵, R⁶, R⁹, R¹⁰, and R¹¹ are each independently a substituted or unsubstituted alkyl, and at least one, preferably two or more, thereof is an alkyl substituted with a water-soluble group. In a preferred embodiment, R⁴, R⁵, R⁶, R⁹, R¹⁰, and R¹¹ are each independently a substituted or unsubstituted alkyl, at least one of R⁴, R⁵, and R⁶ is an alkyl substituted with a water-soluble group, and at least one of R⁹, R¹⁰, and R¹¹ is an alkyl substituted with a water-soluble group.

The term "water-soluble group" in the present specification refers to a group containing one or a plurality of polar and/or ionic substituents, for improving the solubility of the molecule as a whole in an aqueous medium.

Examples of the water-soluble group include, but are not limited to, a carboxylate (-CO₂⁻) group, a sulfonate (-SO₃⁻) group, a sulfonyl (-SO₂⁻) group, a sulfate (-SO₄⁻²) group, a hydroxy (-OH) group, a phosphate (-OPO₃⁻²) group, a phosphonate (-PO₃⁻²) group, an amino (-NH₂) group, and substituted or unsubstituted quaternary nitrogen (each having any counterion). Examples of the counterion include, but are not limited to, sodium, potassium, calcium, ammonium, an organic amino salt, and a magnesium salt. The counterion is preferably a biologically acceptable counterion.

The nitrogen atoms to which R⁴, R⁵, R⁶, R⁹, R¹⁰, and R¹¹ are bonded may each be trivalent or tetravalent.

In one embodiment, each of R¹², R¹³, R¹⁴, R¹⁵, R¹⁶, R¹⁷, R¹⁸, R¹⁹, R²⁰, R²¹, R²², and R²³ is hydrogen.

In one embodiment, X² and X³ are each independently a C₁ to C₁₀ alkylene with or without a heteroatom interposed between the carbon-carbon bonds. In one embodiment, nitrogen added to X² and/or X³ can be quaternized.

In one preferred embodiment, the site containing a phthalocyanine skeleton used in the present invention is a compound represented by formula (Ib): 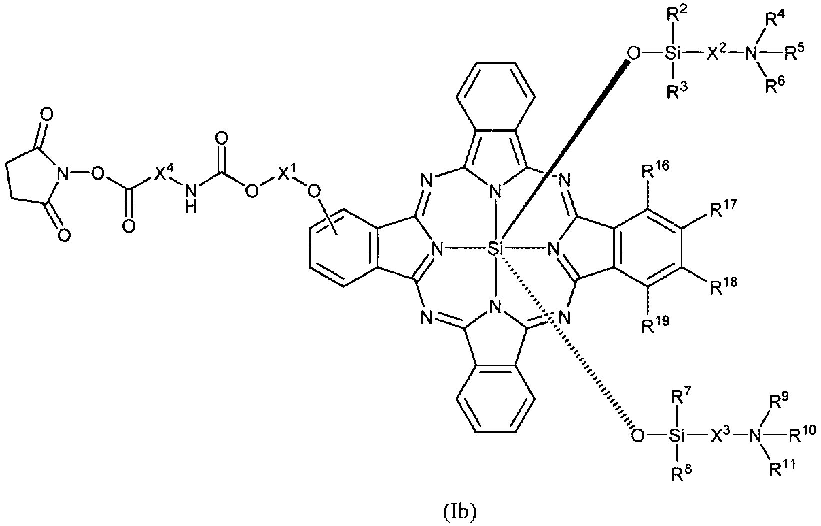 wherein
X¹ and X⁴ are each independently a C₁ to C₁₀ alkylene with or without a heteroatom interposed between the carbon-carbon bonds; and
R², R³, R⁴, R⁵, R⁶, R⁷, R⁸, R⁹, R¹⁰, R¹¹, R¹⁶, R¹⁷, R¹⁸, R¹⁹, X², and X³ are as defined for the above formula (Ia).

In the compound of the above formula (Ib), the reactive group for forming a bond with the target recognition molecule or nanoparticle is an NHS ester. In one embodiment, the reactivity of the NHS ester can be controlled by changing the length of the alkylene group of X⁴ present between the NHS ester and the carbamate functional group. In one embodiment, the length of the alkylene group of X⁴ between the NHS ester and the carbamate functional group is inversely proportional to the reactivity of the NHS ester. In one embodiment, X⁴ is a C₅-alkylene. In another embodiment, X⁴ is a C₃-alkylene. In one embodiment, X¹ is a C₆-alkylene.

In another embodiment, X¹ is a C₃-alkylene.

In one embodiment, the compound of formula (Ia) or formula (Ib) has a net charge of zero. In certain cases, the neutral charge can be obtained with one or a plurality of any counterions, or quaternized nitrogen.

In one embodiment, after the compound of formula (Ia) or formula (Ib) has been bonded to the target recognition molecule or nanoparticle, the molecule or particle retains its solubility, and thus the compound has sufficient solubility in an aqueous solution.

In one preferred embodiment, the site containing a phthalocyanine skeleton used in the present invention is IR700 NHS ester, for example, IRDye 700DX NHS ester (LI-COR Biosciences, P/N929-70010 or 929-70011). In one preferred embodiment, the site containing a phthalocyanine skeleton is a compound represented by the following formula (II).

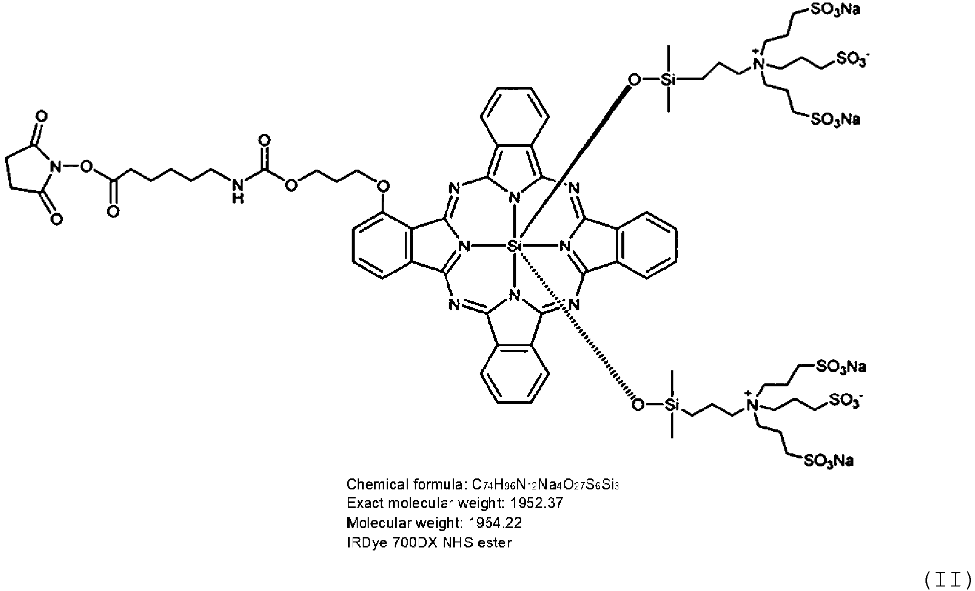

The above-mentioned photosensitive site is preferably contained in the complex of the present invention in a configuration of being bonded to the target recognition molecule or nanoparticle via its reactive group. For example, the compound represented by formula (Ia), (Ib), or (II), "IR700", "IRDye 700DX", or any of modified forms thereof, which may be contained in the complex of the present invention, refers to those compounds in a configuration of being bonded to the target recognition molecule or nanoparticle via its reactive group. In general, IR700 has some preferred chemical characteristics. Amino-reactive IR700, for example, IR700NHS ester or the compound of formula (II), is relatively hydrophilic and can be covalently bonded to the target recognition molecule through use of the NHS ester. IR700 typically has an absorption coefficient (2.1×10⁵ M⁻¹cm⁻¹ in terms of absorption maximum at 689 nm) 5 or more times as high as those of conventional photosensitizers, such as a hematoporphyrin derivative Photofrin (registered trademark) (1.2×10³ M⁻¹cm⁻¹ at 630 nm), meta-tetrahydroxyphenylchlorin; Foscan (registered trademark) (2.2×10⁴ M⁻¹cm⁻¹ at 652 nm), and mono-L-aspartyl chlorin e6; NPe6/Laserphyrin (registered trademark) (4.0×10⁴ M⁻¹cm⁻¹ at 654 nm).

The site containing a phthalocyanine skeleton used in the present invention, for example, the compound represented by the above-mentioned formula (Ia), (Ib), or (II), can be produced using a commercially available starting material. For example, the skeleton is synthesized by fusing two or more different diiminoisoindolines. A synthesis strategy using different dinitriles or diiminoisoindolines can derive phthalocyanines having various degrees of substitution and/or regioisomers having various degrees of distribution. An exemplary synthetic scheme for generating a phthalocyanine skeleton is described in U.S. Patent No. 7,005,518.

In the complex of the present invention, the above-mentioned photosensitive site only needs to be bonded to the target recognition molecule or nanoparticle, but is preferably bonded to the target recognition molecule. The target recognition molecule is preferably an antibody. The complex of the present invention can include one or two or more of the above-mentioned photosensitive sites, and those photosensitive sites can have identical or different structures.

### 1.4. Efficacy

The complex of the present invention is used for CDT of tumor. Specifically, the complex of the present invention exhibits a tumor therapeutic effect by CDT with light irradiation. It is considered that the therapeutic effect of the complex of the present invention by CDT is caused by the Fenton reaction using the iron oxide of the nanoparticle of the complex caused by light irradiation to the complex. Therefore, the CDT by the complex of the present invention is caused by the photo-Fenton reaction.

The complex of the present invention generates heat with respect to light irradiation, for example, irradiation with NIR (for example, light having a wavelength of 500 to 1500 nm), and can exhibit a therapeutic effect by PTT. Since the complex of the present invention contains the nanoparticle which is a magnetic particle containing iron oxide, it enables imaging in vivo.

When the complex of the present invention has photosensitive site, the therapeutic effect by photoimmunotherapy, specifically, NIR-PIT can be exerted. The therapeutic effect of the complex of the present invention by NIR-PIT is considered to be because the photosensitive site of the complex bonded to the tumor cells is hydrophobized by NIR irradiation to cause aggregation of the complex, thereby disrupting cell membranes of the tumor cells and inducing cell death.

Therefore, the complex of the present invention enables combined treatment of CDT and PTT. When the complex of the present invention has photosensitive site, combined treatment of CDT and NIR-PIT, or combined treatment of CDT, NIR-PIT, and PTT is enabled. The complex of the present invention can also be used not only for treatment of a tumor of a patient but also for diagnosis (for example, confirmation of the location of the complex by in vivo imaging using the complex, or imaging of a tumor to which the complex is bound).

### 2. Method for producing complex

The complex of the present invention can be produced by bonding the nanoparticle and the target recognition molecule. The bonding between the target recognition molecule and the nanoparticle can be performed by known means. When the complex of the present invention has photosensitive site, it is preferable to first synthesize target recognition molecule and/or nanoparticle having the photosensitive site. The bonding between the photosensitive site and the target recognition molecule or nanoparticle can be performed by known means.

For example, as a specific method for bonding the photosensitive site and the antibody, there is a method disclosed in Patent Literature 5 or Experiment 1 described later. As a more specific example, it is appropriate that an aqueous phosphate solution containing the antibody and the phthalocyanine compound represented by the above formula (Ia) having an NHS ester in the reactive group Q (for example, the above-mentioned IR700 NHS ester) is incubated at room temperature, and a target antibody-photosensitive site conjugate is purified from the reaction solution by, for example, column purification.

When the photosensitive site is bonded to the nanoparticle, for example, the compound represented by the above formula (Ia) having an NHS ester in the reactive group Q, and the nanoparticle having an amino group or a protein molecule, such as avidin, streptavidin, or protein A, introduced to the surface of the particle can be subjected to a reaction by a method similar to the foregoing.

The number of bonds of the photosensitive site to the target recognition molecule is preferably 1 or more, and more preferably 2 or more, and is preferably 5 or less, and more preferably 4 or less per target recognition molecule (for example, antibody). The number of bonds of the photosensitive site to the nanoparticle is preferably 1 or more, and is preferably 80 or less, more preferably 50 or less, and still more preferably 20 or less per nanoparticle.

The complex of the present invention can be produced by bonding the target recognition molecule and the nanoparticle. For example, by mixing the target recognition molecule that has been biotinylated by a conventional method with the nanoparticle having a substance that binds to biotin introduced to the surface of the particle, the target recognition molecule is bonded to the nanoparticle. Examples of the substance that binds to biotin include proteins, such as avidin and streptavidin. The substance that binds to biotin is introduced at preferably from 1 to 5, still more preferably from 1 to 4, particularly preferably from 1 to 3 molecules per nanoparticle.

Alternatively, the antibody and the nanoparticle can be subjected to a crosslinking reaction by utilizing a carbodiimide compound such as EDC (1-ethyl-3-(3-dimethylaminopropyl)carbodiimide) and an N-hydroxysuccinimide derivative such as Sulfo-NHS. In this case, the loading amounts of the target recognition molecule and the nanoparticle are set so that the number of bonds of the target recognition molecule per nanoparticle is a desired number (preferably 1 or more, and preferably 20 or less, more preferably 16 or less, still more preferably 12 or less, even more preferably 5 or less). The loading amount may be set so that the number of bonds of the target recognition molecule per 1 mg of nanoparticle is a desired number. Specifically, the lower limit of the loading amount of the target recognition molecule per 1 mg of nanoparticle is preferably 1.0×10⁻¹² mol/mg, more preferably 5.0×10⁻¹² mol/mg, and still more preferably 1.0×10⁻¹¹ mol/mg. Meanwhile, the upper limit of the loading amount of the target recognition molecule per 1 mg of nanoparticle is preferably 1.0×10⁻⁸ mol/mg, more preferably 5.0×10⁻⁹ mol/mg, and still more preferably 1.0×10⁻⁹ mol/mg. The bonding between the target recognition molecule and the nanoparticle is not limited to a covalent bond, and may include bonding, for example, by a hydrogen bond, an ionic bond, a hydrophobic interaction, or a combination thereof.

### 3. Composition containing complex

In one embodiment, the present invention provides a composition containing the above-mentioned complex of the present invention. The composition containing the complex of the present invention (hereinafter, also referred to as the composition of the present invention) is used as a pharmaceutical composition for CDT. In a preferred embodiment, the composition of the present invention is used as a pharmaceutical composition for combined treatment of CDT and PTT. When the complex of the present invention has photosensitive site, the composition of the present invention is used as a pharmaceutical composition for combined treatment of CDT and NIR-PIT, or combined treatment of CDT, NIR-PIT, and PTT. In another embodiment, the composition of the present invention is used as a pharmaceutical composition for performing in vivo imaging (for example, tumor imaging) in addition to treatment of a tumor with CDT, or further NIR-PIT and/or PTT.

In one embodiment, the composition of the present invention contains the complex of the present invention and a pharmaceutically acceptable carrier or excipient. Examples of the pharmaceutically acceptable carrier include, but are not limited to, water, oil, a buffer, phosphate buffered saline, and other diluents for injections. Examples of the excipient include, but are not limited to, starch, glucose, lactose, dextrose, carboxymethylcellulose, glycerol, propylene glycol, water, and ethanol. As required, the composition of the present invention can contain a lubricant, a binder, a wetting agent, an emulsifier, a pH adjusting agent, an isotonic agent, a buffer, an antioxidant, a suspending agent, a solubility improver, a preservative, a chelating agent, and other pharmaceutically acceptable substances. For example, the pharmaceutically acceptable carrier and excipient are known in the art (see, for example, Remington's Pharmaceutical Sciences, by E. W. Martin, Mack Publishing Co., Easton, Pa., 19th Edition, 1995).

In one embodiment, the composition of the present invention can contain hydrogen peroxide. The hydrogen peroxide can be contained as a substrate for Fenton reaction, in the composition of the present invention for the purpose of supplementing hydrogen peroxide generated in cells. The hydrogen peroxide can reduce Fe³⁺ to Fe²⁺ to generate oxygen. Fe2⁺ acts as a substrate for Fenton reaction, and it is expected that hyperoxia therapy for cancer is performed by the generated oxygen.

In one embodiment, the composition of the present invention can contain a biological reducing substance. Examples of the biological reducing substance include reduced glutathione, catalase, peroxidase, cytochrome peroxidase, and glutathione peroxidase. The biological reducing substance is used for the purpose of reducing Fe³⁺ to Fe²⁺. The biological reducing substance is used for the above-mentioned purpose because it causes reduction of reactive oxygen species (ROS) or decomposition of hydrogen peroxide.

It is appropriate that the composition of the present invention have a liquid form such as a dispersion; or a solid form such as a powder, a pill, a tablet, a capsule, a transdermal patch, an inhalant, or a suppository. Alternatively, the composition of the present invention may be administered by being reconstituted with a pharmaceutically acceptable carrier (for example, a diluent for an injection) at the time of use. The composition having such a liquid or solid form can be prepared in accordance with a conventional method. In one embodiment, the composition of the present invention is a one-part formulation containing a carrier or excipient containing the complex of the present invention. In another embodiment, the composition of the present invention is a two-part formulation separately containing the complex of the present invention and, for example, a diluent to be administered therewith. Therefore, in one embodiment, the composition of the present invention encompasses a reagent kit containing the complex of the present invention. In one embodiment, the reagent kit contains hydrogen peroxide and/or a biological reducing substance together with the complex of the present invention.

The form of the composition of the present invention may depend on its dosage regimen. The dosage regimen of the composition can be appropriately designed according to, for example, the type and condition of the tumor to be treated, and the type, age, and condition of the patient. The composition may be an oral formulation or a parenteral formulation, and may be, for example, an injectable preparation, an oral preparation, or an external preparation. The composition may be configured to be for single-time administration or for a plurality of times of administration. The content of the complex of the present invention in the composition can be appropriately designed in accordance with, for example, the form of the composition, and its dose for the patient.

The composition of the present invention is adjusted to a pH range compatible with an animal's body, for example, a pH of 5 or more, preferably a pH of 5.5 or more, and is a pH of 10 or less, preferably a pH of 8 or less, more preferably a pH of 7.3 or less, or preferably a pH of 5.5 to 10, more preferably a pH of 5.5 to 8, and still more preferably a pH of 5.5 to 7.3. The pH of the composition can be adjusted with, for example, the above-mentioned pH adjusting agent or a buffer.

### 4. Treatment of a tumor using complex of present invention

As described above, the complex of the present invention or the composition of the present invention containing the complex is used for the treatment of a tumor with CDT. In one embodiment, the present invention provides a method for treating a tumor with CDT using the complex or composition of the present invention. The method for treating a tumor according to the present invention (hereinafter, also referred to as the treatment method of the present invention) includes a step of administering the complex or composition of the present invention to a patient, and a step of irradiating the patient with near infrared rays (NIR). Note that, in the present specification, the "method for treating a tumor" can be read as a "method for killing a tumor".

As described above, the complex and the composition of the present invention enable combined treatment of CDT and PTT, and when the complex of the present invention has photosensitive site, combined treatment of CDT, PTT, and NIR-PIT is enabled. Therefore, in one embodiment, the method for treating a tumor using the complex and composition of the present invention is a method for treating a tumor by a combination of CDT and PTT. In another embodiment, the method for treating a tumor using the complex and composition of the present invention is a method for treating a tumor by using CDT and NIR-PIT in combination, or CDT, NIR-PIT, and PTT in combination.

The patient to which the complex or composition of the present invention is administered is a patient in need of CDT for the treatment of a tumor. In one embodiment, the patient is a patient in need of combined treatment of CDT and NIR-PIT for the treatment of a tumor. Examples of the patient include a human and a non-human animal each having a tumor to be treated with CDT. Examples of the non-human animal include, but are not limited to, non-human mammals such as a mouse, a rat, a hamster, a rabbit, a pig, a goat, a dog, a cat, a sheep, a bovine, and a horse. The patient may or may not have the experience of another treatment for a tumor (for example, surgery, radiation therapy, phototherapy, and chemotherapy using an agent other than the complex or composition of the present invention).

The target recognition molecule contained in the complex of the present invention is selected according to the type of the tumor to be treated. Appropriate selection of target recognition molecules allows accumulation of the complexes of the present invention into the tumor to be treated. Preferably, the target recognition molecule is an antibody and can specifically bind to an antigen present on the surface of the tumor cell, preferably a tumor-specific protein expressed on the surface of the tumor cell. A cell surface antigen of the tumor, for example, a tumor-specific protein, to be targeted by the target recognition molecule can be determined in accordance with known information. A person skilled in the art can select an antibody specific for a target antigen on the tumor to be treated.

### 4.1. Administration of complex

The dosage regimen (such as administration route, dose, and number of times) of the complex or composition of the present invention can be appropriately determined in accordance with, for example, the type and condition of the tumor to be treated and the type, age, and condition of the patient. Examples of the administration route include local administration to a tumor-affected area via, for example, injection, a catheter, spraying, application, a patch, or a suppository, and systemic administration via, for example, infusion, oral administration, intraperitoneal administration, or intravenous injection. Preferably, the complex or composition of the present invention is locally administered. In one embodiment, the complex or composition of the present invention is intravenously administered. In one embodiment, the complex or composition of the present invention is directly administered to the affected area of the tumor through use of, for example, a syringe, or is injected via a catheter. The complex or composition of the present invention may be used alone for the treatment for a tumor, but may be also used in combination with another agent or therapy, such as surgery, radiation therapy, phototherapy, and chemotherapy using an agent other than the complex or composition of the present invention.

The complex or composition of the present invention only needs to be administered in an effective amount to the patient. The "effective amount" refers to an amount allowing the complex of the present invention to accumulate in a tumor to be treated in a patient in an amount sufficient for exhibiting the therapeutic effect on the tumor. The "effective amount" preferably refers to such an amount that, while exhibiting the therapeutic effect on a tumor to be treated in a patient, side effects on the patient can be minimized or suppressed within acceptable ranges.

The dosage of the complex or composition of the present invention to the patient can be appropriately determined depending on, for example, the type and condition (such as location or volume) of a tumor to be targeted, the type, age, condition, and administration route of the patient, and the form of the composition containing the complex. For example, the dosage of the complex of the present invention can be set in accordance with a tumor volume. The tumor volume (V) can be calculated by, for example, measuring the minor axis (W) and major axis (L) of the tumor and substituting the measured values into the equation: V = (W² × L)/2. Alternatively, the dosage can be adjusted in accordance with the degree of accumulation of the complex of the present invention in the tumor, which is measured by imaging described later. The dosage of the complex of the present invention for a human can be determined based on its dosage for a mouse. For example, when the complex of the present invention is administered to a human, its effective amount can be determined to be an amount that is from 5 to 10 times its effective amount in a mouse.

In one embodiment, the single dosage of the complex or composition of the present invention may be in a range of 0.01 mg to 9000 mg as the amount of the complex of the present invention, for example in the case of topical infusion. In one embodiment, the single dosage of the composition of the present invention may be, for example, 0.5 mL to 1000 mL in the case of an injectable formulation. In one embodiment, the composition of the present invention is an injectable formulation, the single dosage thereof is 1 to 5 mL, and the composition contains 0.1 mg to 5000 mg of the complex of the present invention in the single dosage.

In one embodiment, when the complex or composition of the present invention is injected into a tumor-affected area of an adult (60 kg), the single dosage (injection amount) thereof is 0.01 mg to 20 mg/kg (body weight) as the amount of the complex of the present invention. In another embodiment, when the composition of the present invention is injected into a tumor-affected area of an adult (60 kg), the single dosage (injection amount) thereof is generally 1 to 5 mL.

The dosage of the complex or composition of the present invention and the number of times of administration thereof can be increased or decreased in accordance with the therapeutic effect on the tumor. The therapeutic effect on the tumor can be evaluated by a general evaluation method for tumor treatment, such as the shrinkage rate of a tumor tissue. In one embodiment, the complex or composition of the present invention is administered a single time in the above-mentioned dosage. In another embodiment, the complex or composition of the present invention is administered a plurality of times.

In the case of a plurality of times of administration, the above-mentioned dosage may be repeatedly administered, or the dosage may be increased or decreased in accordance with the therapeutic effect on the tumor. In one embodiment, second or subsequent administration can be carried out after the clearance of the dose of the last administration from the patient. In another embodiment, the complex or composition of the present invention can be repeatedly administered once in 1 week, once in 2 weeks, once in 1 month, or at a lower frequency. In another embodiment, the complex or composition of the present invention can be administered again when the tumor to be treated remains after 1 week, 2 weeks, 3 weeks, 4 weeks, 2 months, 6 months, 1 year, or more from the last administration.

In a preferred embodiment, in the treatment method of the present invention, hydrogen peroxide is further administered to the patient in addition to the complex of the present invention. As hydrogen peroxide, for example, hydrogen peroxide is administered to the patient by drip infusion. Alternatively, the composition of the present invention containing hydrogen peroxide as described above may be administered to the patient. In another preferred embodiment, in the treatment method of the present invention, a biological reducing substance is further administered to the patient in addition to the complex of the present invention. Examples of the biological reducing substance are as described above. Alternatively, the composition of the present invention containing a biological reducing substance as described above may be administered to the patient. The hydrogen peroxide and the biological reducing substance may be used in combination.

The administration route of the hydrogen peroxide and the biological reducing substance and the number of times of administration thereof can be appropriately determined in the same manner as in the complex or composition of the present invention. For example, hydrogen peroxide or a biological reducing substance can be administered to the patient together with the complex or composition of the present invention. The dosage of the hydrogen peroxide and the biological reducing substance can be appropriately set according to the patient. Preferably, the dosage of the hydrogen peroxide and the biological reducing substance is set to such an amount that the therapeutic effect on the tumor by the complex or composition of the present invention is enhanced, and side effects caused by the administration of the hydrogen peroxide and the biological reducing substance to the patient can be minimized or within acceptable ranges.

### 4.2. Light irradiation

Subsequent to the administration of the complex or composition of the present invention to a patient, the patient is irradiated with light. Preferably, the patient is irradiated with NIR. Preferably, the tumor cell or tumor-affected area to which the complex of the present invention is bound is locally irradiated with NIR. The light-irradiated complex of the present invention exerts the therapeutic effect on the tumor by CDT, or further exerts the therapeutic effect on the tumor by PTT. When the complex of the present invention has photosensitive site, the NIR-irradiated complex further exerts the therapeutic effect on the tumor by NIR-PIT. That is, the NIR-irradiated complex causes a photochemical reaction and kills the tumor cell to which the complex is bound.

The wavelength of the light beam to be radiated is preferably 500 to 1500 nm, more preferably 600 to 850 nm, and still more preferably 660 to 740 nm. The timing of the irradiation can be determined to be any timing after the administration of the complex of the present invention. For example, the timing can be determined to be any timing between 30 minutes and 96 hours after the administration, preferably between 30 minutes and 48 hours, between 30 minutes and 24 hours, between 1 hour and 48 hours, or between 1 hour and 24 hours after the administration. The period of time of the irradiation can be appropriately determined in a range of 5 seconds to 72 hours. The irradiation can be performed once or a plurality of times so that the cumulative irradiation time per administration of the complex of the present invention falls within the above range. The period of time per irradiation can be appropriately determined, and can be set to, for example, 5, 10, 15, 20, 25, 30, 35, 40, 45, 50, or 55 seconds, or 1, 1.5, 2, 2.5, 3, 3.5, 4, 4.5, or 5 minutes, or 10, 20, 30, 40, 50, or 60 minutes, or 1 or 2 hours.

In one embodiment, the dose of light with which the patient is irradiated is preferably 1 J/cm² or more, more preferably 5 J/cm² or more, and still more preferably 10 J/cm² or more, is preferably 1000 J/cm² or less, more preferably 500 J/cm² or less, still more preferably 100 J/cm² or less, and still more preferably 50 J/cm² or less, and for example, falls within a range of 1 to 1000 J/cm², 1 to 500 J/cm², 5 to 200 J/cm², 10 to 100 J/cm², or 10 to 50 J/cm².

The irradiation can be carried out once or a plurality of times with respect to the administration of the complex of the present invention performed once. Therefore, the irradiation may be completed at one time, or may be repeatedly performed over several days. When the irradiation is performed a plurality of times, the conditions for the respective times of irradiation may be identical to or different from each other. The dose, conditions, or method for the irradiation can be changed in accordance with the type and condition of the tumor.

### 4.3. Combination therapy

The above-described light irradiation causes the complex of the present invention to exert the therapeutic effect on a tumor by PTT. That is, the irradiation of the patient with light causes the complex of the present invention to generate heat, and the heat kills tumor cells. Therefore, in a preferred embodiment, the treatment method of the present invention is a method for treating a tumor by combination of CDT and PTT, using the complex or composition of the present invention. When the complex of the present invention has photosensitive site, the above-described light irradiation causes the complex of the present invention to exert the therapeutic effect on a tumor by NIR-PIT. That is, NIR irradiation to the patient causes aggregation of the complex by hydrophobizing the photosensitive site of the complex bonded to the tumor cell, and as a result, the cell membrane of the tumor cell is disrupted to kill the tumor cell. Therefore, in a preferred embodiment, the treatment method of the present invention is a method for treating a tumor by combination of CDT and NIR-PIT or combination of CDT, NIR-PIT, and PTT, using the complex or composition of the present invention.

The complex of the present invention administered to the patient specifically binds to the tumor cell to be treated via the target recognition molecule. The complex of the present invention specifically bonded to a tumor cell kills the bonded tumor cell by the CDT or NIR-PIT and/or PTT mechanism described above. Thus, the present invention provides for selective killing of the tumor cells to be treated.

### 4.4. Imaging

Since the nanoparticle contained in the complex of the present invention is a magnetic particle containing iron oxide, it is possible to perform in vivo imaging by MRI. In one embodiment, the complex or composition of the present invention is used for imaging of a patient or a tumor.

For example, the complex or composition of the present invention administered to the patient is used for tumor imaging before treatment of the tumor with CDT. The tumor imaging enables the recognition of the location of the complex in the body of the patient, or the location of the tumor to which the complex has been bonded, that is, enables the diagnosis of the patient. Thereby, the timing of light irradiation for CDT, or further NIR-PIT and/or PTT, a three-dimensional irradiation position in a living body, and an irradiation dose (irradiation time and dose) can be optimized, and the tumor therapeutic effect of the complex of the present invention can be enhanced. The imaging of a patient or a tumor by MRI can be performed by a conventional method.

### 4.5. Other methods

The techniques for CDT and imaging each using the complex or composition of the present invention described above are applicable not only in vivo, but also in vitro. For example, not only a tumor present in a patient's body, but also cultured tumor cells or a cultured tissue containing tumor cells can be subjected to the administration of the complex or composition of the present invention and light irradiation to reduce the tumor cells or inhibit the growth thereof. The administration method of the complex or composition of the present invention and the conditions of light irradiation can be appropriately changed according to the state of the target cell or tissue. For example, the complex or composition of the present invention can be directly administered to tumor cells in a culture. Milder conditions can be selected for the dosage of the complex or composition, or the conditions for light irradiation as compared to the case of the administration or irradiation for a patient.

### Examples

Hereinafter, the present invention will be described in more detail by way of Examples. However, the present invention is not limited at all by these Examples and the like.

Experiment 1: Production of antibody-nanoparticle complex Nanomag-D-Spio-Pan-IR700

### 1) Production of Pan-IR700

2 mg of a human-type monoclonal antibody panitumumab (hereinafter, also referred to as "Pan") was incubated together with 133.6 µg of IRDye 700DX NHS Ester (hereinafter, also referred to as "IR700", manufactured by LI-COR Biosciences) in 0.2 mol/L of Na₂HPO₄ (pH 8.5) at room temperature for 30 to 120 minutes. The mixture was purified with a Sephadex G50 column (PD-10; GE Healthcare, Piscataway, NJ). The protein concentration was determined with a Coomassie Plus protein assay kit (Pierce Biotechnology, Rockford, IL) by measuring absorption at 595 nm with an ultraviolet-visible system (8453 Value system; Agilent Technologies, Palo Alto, CA). The concentration of IR700 was measured based on absorption according to an ultraviolet-visible system (Shimadzu UV-VIS). The number of IR700 was about 3 molecules per molecule of Pan. Hereinafter, Pan to which IR700 is bonded is referred to as Pan-IR700.

### 2) Biotinylation of Pan-IR700

5.69 mg of (+)-biotin N-hydroxysuccinimide ester (manufactured by Sigma-Aldrich, hereinafter also referred to as Biotin-NHS) was dissolved in 1 mL of DMSO (manufactured by Sigma-Aldrich).

1 mL of a Pan-IR700 solution (2.0 mg/mL) was collected in a microtube, 8 µL of the previously prepared Biotin-NHS DMSO solution ([Biotin-NHS]/[Pan-IR700] = 10) was added, and the mixture was left to stand still at room temperature for 3 hours. Unreacted biotin was removed through an ultrafiltration filter (Amicon Ultra 100k), and the residue was adjusted to 1.9 mg/mL with Dulbecco's Phosphate Buffered Saline (manufactured by Wako Pure Chemical Industries, Ltd., hereinafter also referred to as D-PBS) to obtain biotinylated Pan-IR700. Hereinafter, the biotinylated Pan-IR700 is referred to as Pan-IR700-Biotin.

### 3) Production of complex of Pan-IR700 and magnetic nanoparticle

120 mg (5 mg/mL, 24 mL) of Nanomag-D-Spio 79-19-201 (manufactured by Micromod, streptavidin surface-modified magnetic particles, particle diameter: 20 nm, hereinafter, also referred to as "Nanomag-D-Spio") was collected in a 50 mL tube, 1.8 mg (1.9 mg/mL, 947 µL) of Pan-IR700-Biotin was added, and the mixture was stirred at room temperature for 60 minutes. 18 µg (0.1 mg/mL, 180 µL) of biotin (manufactured by Wako Pure Chemical Industries, Ltd.) was added thereto, and the mixture was stirred at room temperature for 30 minutes. In this way, a dispersion containing an antibody-nanoparticle complex in which Pan-IR700-Biotin and Nanomag-D-Spio are bonded (also referred to as "Nanomag-D-Spio-pan-IR700") was obtained. 1 mL of the above-mentioned dispersion was passed through a column (MS-columns, manufactured by Miltenyi Biotec) placed on a magnetic stand. Thereafter, 2 mL of D-PBS was passed through the column. When the filtrate was irradiated with excitation light having a wavelength of 676 nm and fluorescence measurement was performed at 700 nm, it was confirmed that unreacted Pan-IR700-Biotin was not detected.

### 4) Production of antibody-nanoparticle complex Nanomag-D-Spio-Cont

An antibody-nanoparticle complex (Nanomag-D-spio-cont-IR700) was produced by the same procedure as in 1) to 3) above using a ligand of a tyrosine kinase type receptor not expressed in MDAMB468 and A431 instead of Pan.

### 5) Production of antibody-nanoparticle complex Nanomag-D-Spio-Pan

An antibody-nanoparticle complex (Nanomag-D-Spio-Pan) was produced by the same procedure as in 3) except that biotinylated Pan (Pan-Biotin) obtained by the same procedure as in 2) above was used instead of Pan-IR700-Biotin.

### Experiment 2: Evaluation of Nanomag-D-Spio-pan-IR700

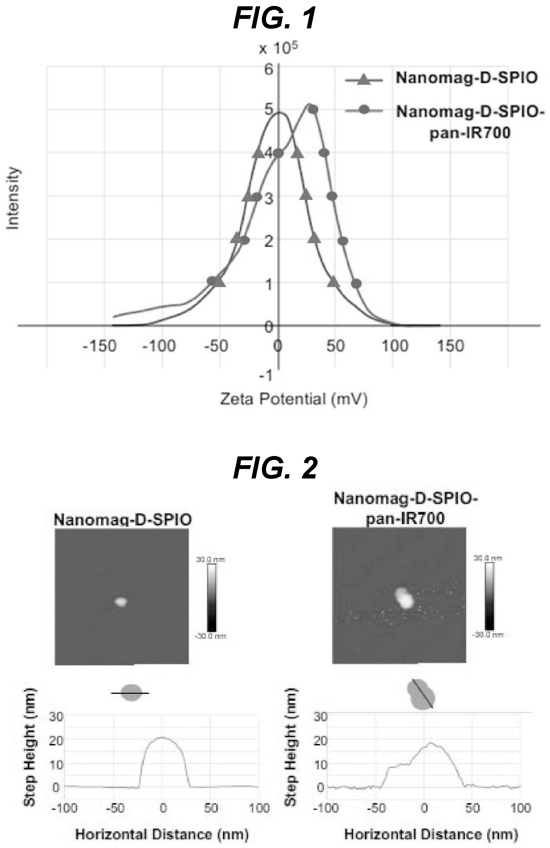

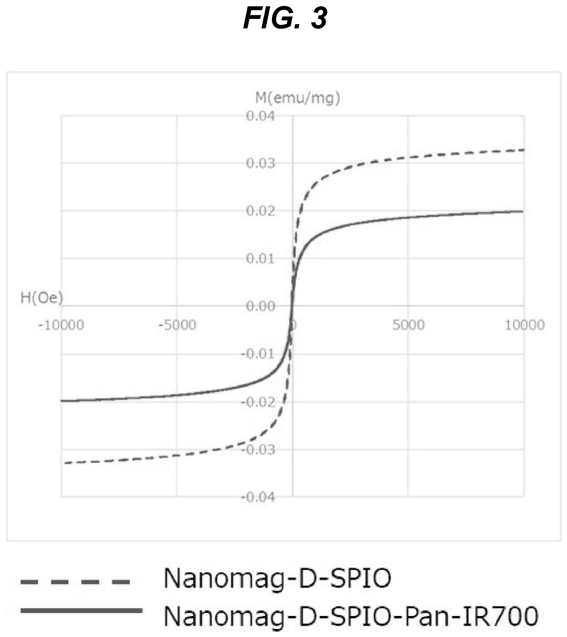

For the antibody-nanoparticle complex Nanomag-D-Spiopan-IR700 produced in Experiment 1 and the nanoparticle Nanomag-D-Spio as a material, zeta potential measurement, atomic force microscope (AFM) observation, and vibrating sample magnetometer (VSM) measurement were performed. For the zeta potential, a dispersion of Nanomag-D-Spio-pan-IR700 and Nanomag-D-Spio was diluted (0.1 mg/ml KCl, pH 7, 25°C), and the zeta potential was measured by Zetasizer^{™} (Malvern Panalytical Ltd). The measurement results of zeta potential are shown in Fig. 1. The AFM image, and the size measurement result and the estimated shape based on the AFM image are shown in Fig. 2. The results of VSM measurement are shown in Fig. 3. From the AFM image, Nanomag-D-Spio was spherical, whereas Nanomag-D-Spio-pan-IR700 had a rough shape.

### Experiment 3: Property of Nanomag-D-Spio-pan-IR700 for binding to cell

The property of the antibody-nanoparticle complex produced in Experiment 1 for binding to cells was evaluated. MDAMB468 and A431 as disease model cells and H661 and 3T3 as normal cells were used as cells. MDAMB468 was human breast cancer cells expressing EGFR, A431 was human epidermoid cancer cells expressing EGFR, H661 was human lung epithelial-like cells not expressing EGFR, and 3T3 was mouse embryonic fibroblasts not expressing EGFR. As a medium for cell culture, RPMI1640 (basic medium) containing 10% fetal bovine serum and 1% penicillin/streptomycin was used. Each cell was seeded on a culture plate (1×10⁵ cells/well) and incubated (37°C, 5 vol% CO₂) for 24 hours. Then, the complex (pan-IR700 or Nanomag-D-Spio-pan-IR700, 10 µg/mL) was added to the well and incubated (37°C, 5 vol% CO₂) for 6 hours. Complex-free cells were taken as control. The fluorescence intensity of the cells after incubation was measured by flow cytometry using IR700 (wavelength: 698 nm) as a fluorescent label.

The measurement results of the fluorescence intensity are shown in Fig. 4(A). In Fig. 4(A), "Pan blocking" indicates the fluorescence intensity of cells incubated in the presence of an EGFR inhibitor (panitumumab, tyrosine kinase inhibitor), and here, the cells were seeded in a culture plate in the same manner as described above, and then incubated (37°C, 5 vol% CO₂) for 24 hours, then the EGFR inhibitor was placed in a well, and the complex was placed in the well and incubated (37°C, 5 vol% CO₂) for 6 hours. As shown in Fig. 4(A), it was suggested that the EGFR positive cells MDAMB468 and A431 specifically bind to pan-IR700 or Nanomag-D-Spio-pan-IR700.

Instead of pan, MDAMB468 and A431 were cultured in the presence of a complex using an antibody (IgG antibody)-nanoparticle complex (Nanomag-D-spio-cont-IR700) to which a ligand of a tyrosine kinase type receptor not expressed in MDAMB468 and A431 was bonded, in the same procedure as described above, and the fluorescence intensity of the cells was measured by flow cytometry. The measurement results of the fluorescence intensity are shown in Fig. 4(B). As shown in Fig. 4(B), the fluorescence intensity of Nanomag-D-spio-cont-IR700 was not different from the control. From the results in Fig. 4(B), it was suggested that Nanomag-D-Spio-cont-IR700 is not bonded to MDAMB468 and A431.

### Experiment 4: Stability of Nanomag-D-Spio-pan-IR700 in blood

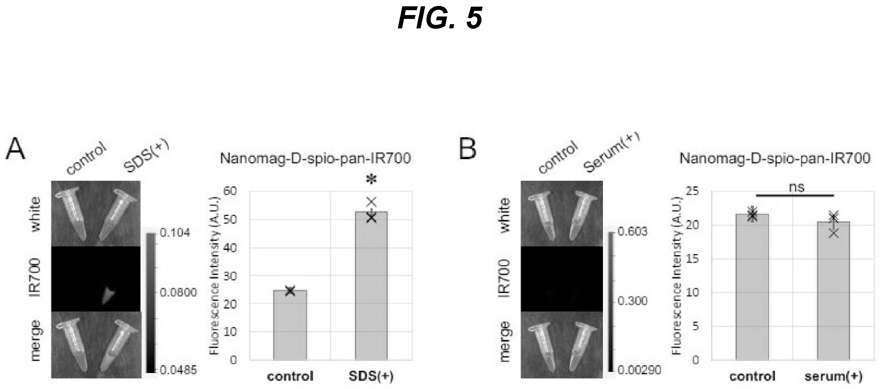

In a microtube, 100 mL of a Nanomag-D-Spiopan-IR700 solution (5 µg/mL) diluted with PBS and 100 mL of a PBS diluted sodium lauryl sulfate (SDS) solution (1 wt%) or 400 mL of mouse serum were added and incubated for 6 hours. Using IR700 (wavelength: 698 nm) as a fluorescent label, the fluorescence intensity of the solution after incubation was measured. The Nanomag-D-Spio-pan-IR700 solution without SDS or serum was used as a control. The measurement results are shown in Figs. 5(A) and 5(B).

The intensity of fluorescence derived from Nanomag-D-Spio-pan-IR700 was about 2.2 times the control in SDS, but there was no difference from the control in serum.

### Experiment 5: Incorporation of Nanomag-D-Spio-pan-IR700 by endocytosis

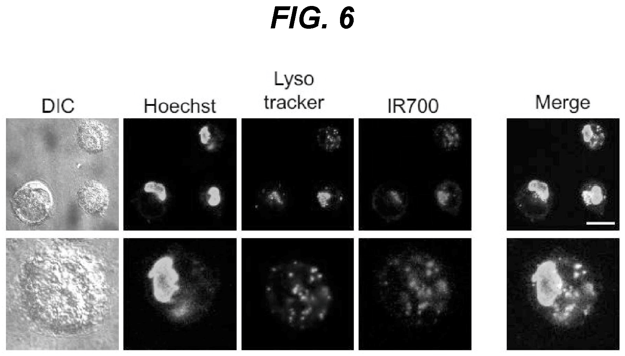

A431 cells were seeded in a culture plate (1×10⁵ cells/well, culture medium was the same as in Experiment 3) and incubated for 24 hours (37°C, 5 vol% CO₂). Nanomag-D-Spio-pan-IR700 was added to the well so as to be 10 µg/mL, and cultured and incubated for 6 hours (37°C, 5 vol% CO₂), and then the cells were fluorescently stained (Hoechst staining, lysosome staining (green)) and subjected to fluorescence observation. Fig. 6 shows fluorescence images. In Fig. 6, "DIC" represents a bright field image by an optical microscope, "Hoechst", "Lyso tracker", and "IR700" represent fluorescence images of labels with Hoechst staining, lysosome staining, and IR700, and "Merge" represents a superimposed image of each fluorescence image.

### Experiment 6: Reaction of nanoparticle with methylene blue

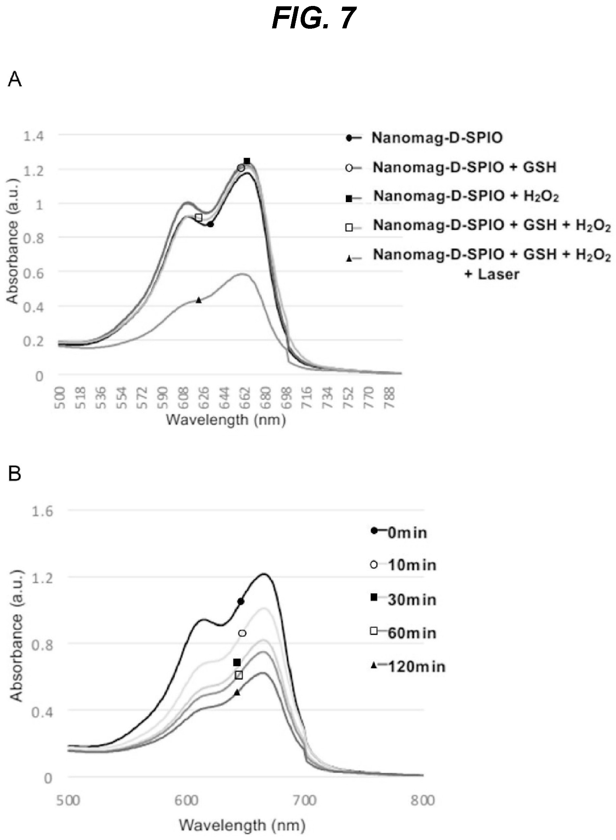

In a microtube, Nanomag-D-Spio (0.5 mg/mL) was added and heated at 37°C for 30 minutes in the presence or absence of reduced glutathione (GSH, 10 mM). Methylene blue (10 µg/mL) was added to the heated tube with or without hydrogen peroxide (1 mM). The solution in the microtube was adjusted to pH 5.4 with PBS containing HCl and the absorbance (500 to 800 nm) was measured. The solution was irradiated with laser light (690 nm, 200 J/cm², 2 hours), and the absorbance (500 to 800 nm) was measured. The measurement results of absorbance are shown in Fig. 7(A).

Similarly, in a microtube, Nanomag-D-Spio (20 µg/mL) and reduced glutathione (10 mM) were added and heated at 37°C for 0 to 120 minutes, and then methylene blue (10 µg/mL) and hydrogen peroxide (20 µM) were added thereto. The solution in the microtube was adjusted to pH 5.4 with PBS containing HCl and the absorbance (500 to 800 nm) was measured. The results are shown in Fig. 7(B).

### Experiment 7: Exothermic behavior of antibody-nanoparticle complex by near infrared rays

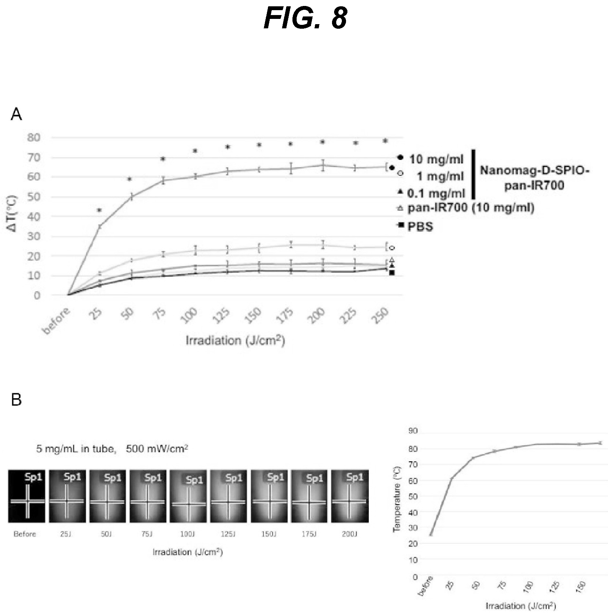

In a microtube, a liquid containing Nanomag-D-Spio-pan-IR700 (0.1 to 10 mg/mL) or pan-IR700 (10 mg/mL) was added, and irradiated with laser light (690 nm, 500 mW/cm²), and the liquid temperature in the microtube was measured. The temperature rise curve of a complex-containing liquid with respect to the irradiation amount of each liquid is shown in Fig. 8(A). The infrared thermographic image of the Nanomag-D-Spio-pan-IR700-containing liquid after laser irradiation and the liquid temperature measured on the basis of thermography are shown in Fig. 8(B).

Note that, when power conversion efficiency (PCE) by laser irradiation was measured, PCE was 29.21%. From these results, it was shown that the nanoparticle contained in the antibody-nanoparticle complex generates heat when irradiated with near infrared rays.

### Experiment 8: In vitro antitumor activity of antibody-nanoparticle complex

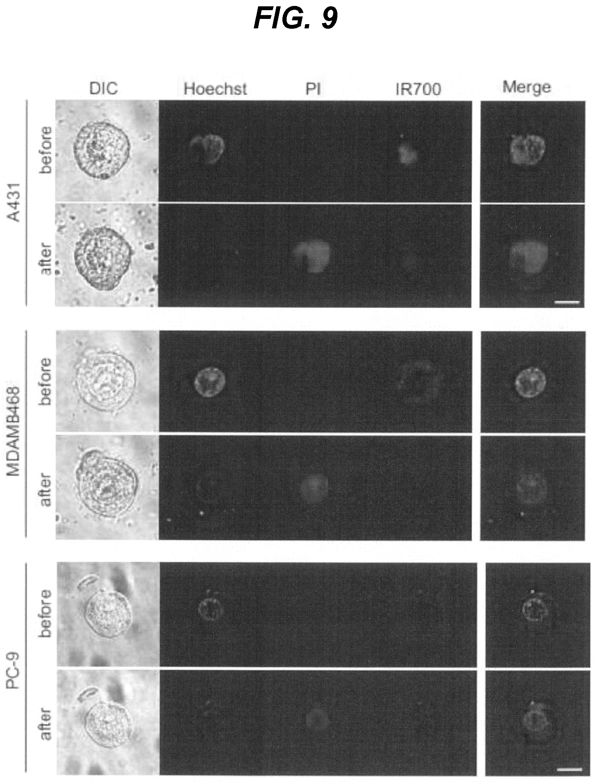

A431, MDAMB468, and PC-9 were used as disease model cells. A431 and MDAMB468 were disease model cells as described in Experiment 3, and PC-9 was a human lung adenocarcinoma cell expressing EGFR. Cells were seeded in a culture plate (1×10⁵ cells/well, culture medium was the same as in Experiment 3) and incubated for 24 hours (37°C, 5 vol% CO₂). Nanomag-D-Spio-pan-IR700 was added to the well so as to be 10 µg/mL, and incubated (37°C, 5 vol% CO₂) for 24 hours. The medium was replaced with PBS and irradiated with laser light (690 nm, 4 mJ/cm²). The cells were fluorescently stained (Hoechst staining, pyridinium iodide (PI) staining) before and after laser irradiation and subjected to fluorescence observation. Fig. 9 shows fluorescence images. In Fig. 9, "DIC" represents a bright field image by an optical microscope, "Hoechst" and "PI" represent fluorescence images of Hoechst staining and PI staining, and "Merge" represents a superimposed image of each fluorescence image.

### Experiment 9: Antitumor activity of antibody-nanoparticle complex against in vitro spheroids

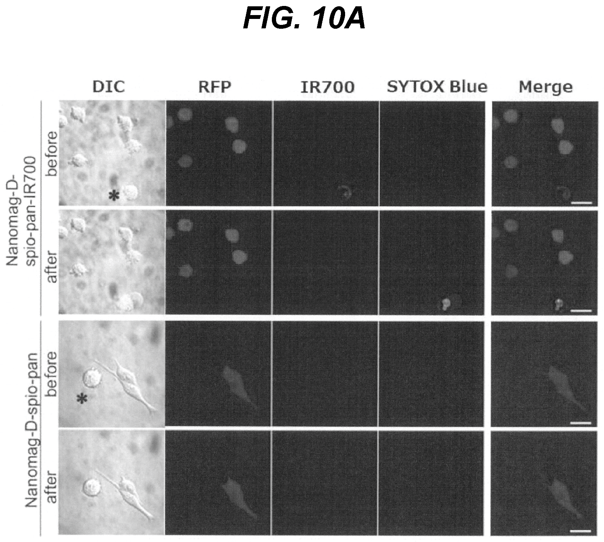

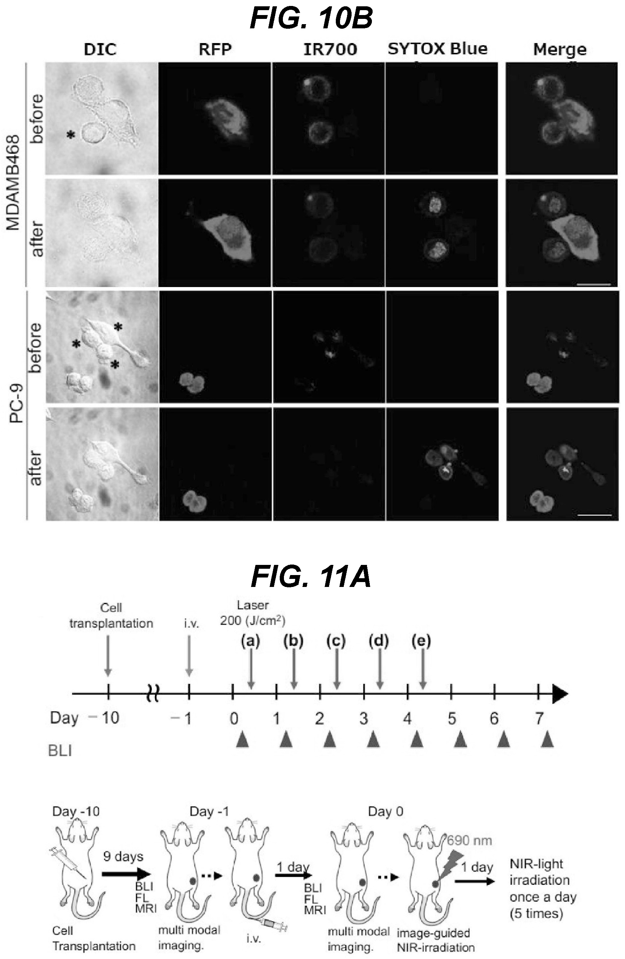

Cells for laser light irradiation were prepared in the same procedure as in Experiment 8 except that spheroids prepared by mixing antigen-presenting cells (A431, MDAMB468, or PC-9) and antigen-non-presenting cells (3T3-RFP) were used instead of the disease model cells. A431, MDAMB468, and PC-9 are the same disease model cells as those used in Experiment 8, and 3T3-RFP is cells obtained by transfecting 3T3 used in Experiment 3 with a gene of red fluorescent protein (RFP) as a reporter gene. The spheroids before and after laser light irradiation were fluorescently stained (SYTOX^{™} Blue staining) and subjected to fluorescence observation. Figs. 10A and 10B show fluorescence images. Fig. 10A shows fluorescence images of spheroids obtained by mixing A431 and 3T3-RFP at 1/1 (volume ratio). Fig. 10B shows fluorescence images of spheroids obtained by mixing MDAMB468 and 3T3-RFP at 1/1 (volume ratio) and spheroids obtained by PC-9 and 3T3-RFP at 1/1 (volume ratio). In the figure, "DIC" represents a bright field image by an optical microscope, "RFP" represents a fluorescence image using a reporter RFP incorporated into 3T3-RFP as a fluorescent label, "IR700" represents a fluorescence image using IR700 as a fluorescent label, "SYTOX Blue" represents a fluorescence image of SYTOX^{™} Blue staining, and "Merge" represents a superimposed image of each fluorescence image.

It was shown that Nanomab-D-Spio-IR700 was bonded only to tumor cells, and fluorescence from IR700 was quenched after laser light irradiation, while cell death of tumor cells to which Nanomab-D-Spio-IR700 was bonded occurred.

### Experiment 10: In vivo antitumor activity of antibody-nanoparticle complex

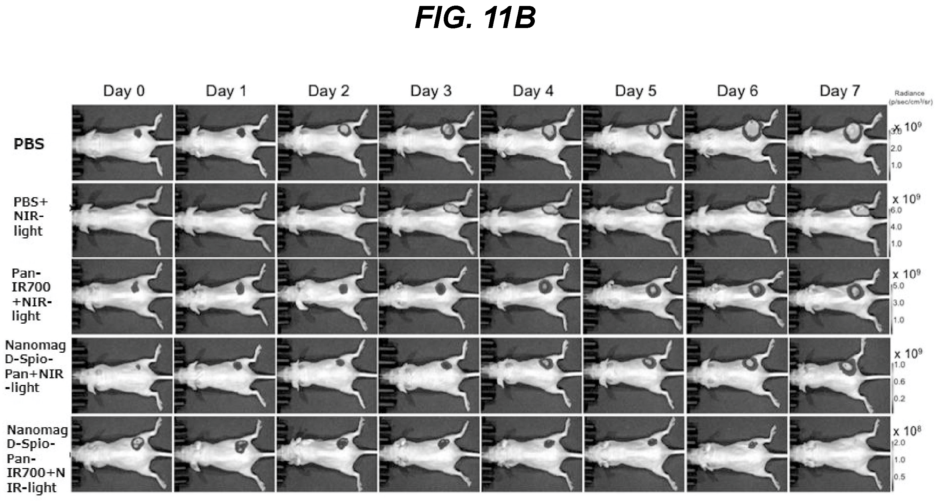

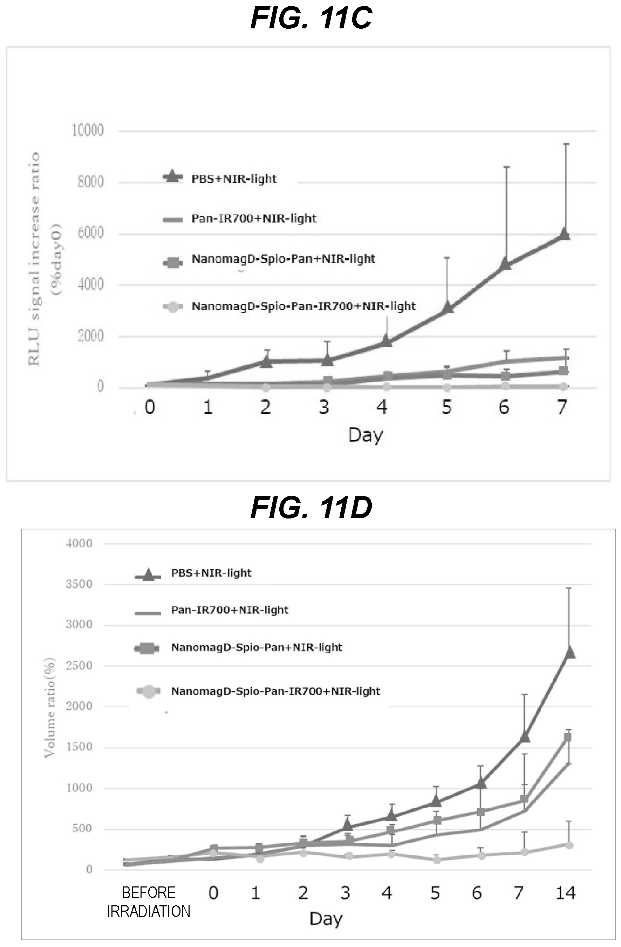

The in vivo antitumor activity of the antibody-nanoparticle complex was evaluated according to experimental protocol shown in Fig. 11A. Disease model cells (A431, 6×10⁶ cell/100 µL) were subcutaneously injected near the root of the right foot of female homozygous athymic nude mice (Day-10 in Fig. 11A) to prepare xenograft model mice. The mice were anesthetized, and PBS or the complex (as a control, pan-IR700, Nanomag-D-Spio-pan, or Nanomag-D-Spio-pan-IR700) was administered into the tail vein at 80 µL/body (30 µg/body in terms of antibody) (Day-1 in Fig. 11A). From the next day (Day 0 in Fig. 11A), the affected area was irradiated with laser light once a day for a total of five times (690 nm, 200 mJ/cm²/time, (a) to (e) in Fig. 11(A)). The size of the tumor after irradiation was evaluated by luciferase assay (Bioluminescent Imaging; BLI) at the timing indicated by "BLI" (arrow in the figure) in Fig. 11A. Fig. 11B shows images of the xenograft model mice observed by the luciferase assay. Fig. 11C is a graph showing a change in tumor size evaluated on the basis of the luciferase assay images (n = 6), in which the change in tumor size is expressed as a relative light unit (RLU) ratio with the RLU in the region of interest of the tumor image on Day 0 set to 100. Fig. 11D is a graph showing a change in tumor volume measured after tumor resection (n = 10). All complexes showed a tendency to reduce the tumor volume in mice irradiated with laser light after complex administration, among which the reduction in tumor volume in Nanomag-D-Spio-pan-IR700-administered mice was significant (Dunnet's test with Pan-IR700+NIR-light as a comparative group, p < 0.05).

### Experiment 11: Antitumor activity of antibody-nanoparticle complex against in vivo spheroids

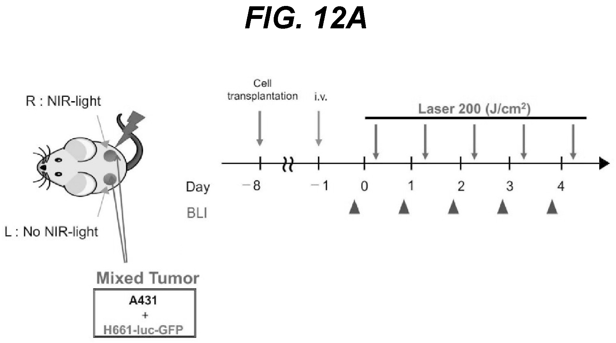

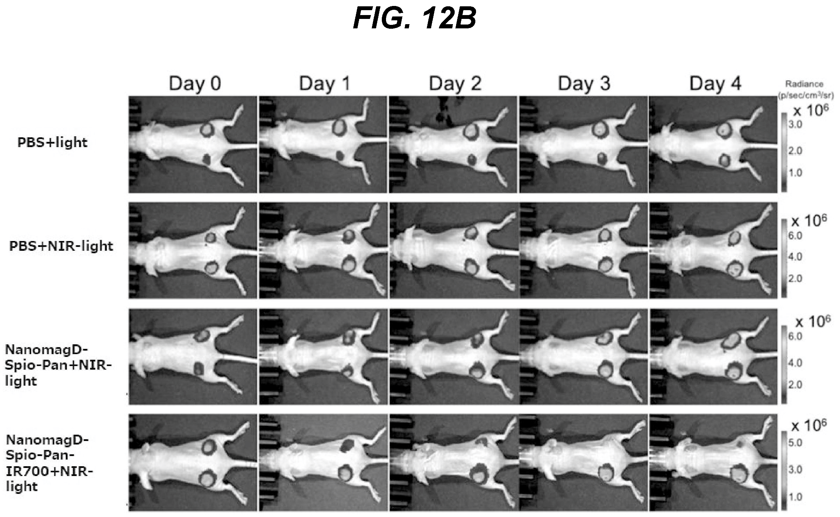

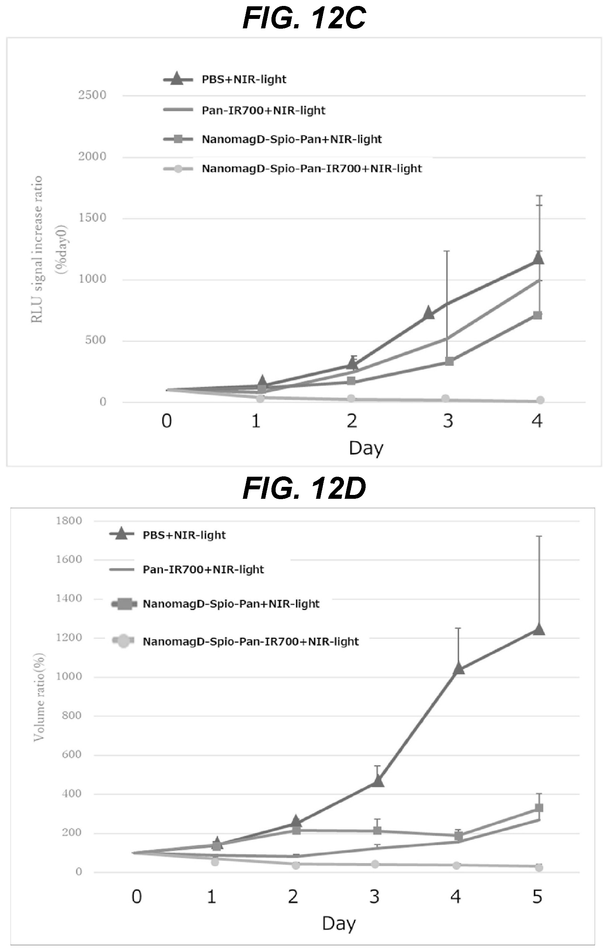

The antitumor activity against in vivo spheroids of the antibody-nanoparticle complex was evaluated according to the experimental protocol shown in Fig. 12A. Spheroids (6×10⁶ cell/100 µL) were prepared by mixing antigen-presenting cells (A431) and antigen-non-presenting cells (H661-luc-GFP) at 1/1 (volume ratio). A431 is the same disease model cells as those used in Experiment 3, and H661-luc-GFP is cells obtained by transfecting H661 used in Experiment 3 with a luciferase gene and a green fluorescent protein (GFP) gene as reporter genes. The prepared spheroids were subcutaneously injected into the vicinity of the two groins on the right and left of female homozygous athymic nude mice (in Fig. 12A, Day-8) to prepare xenograft model mice. The mice were anesthetized, and PBS or the complex (as a control, pan-IR700, Nanomag-D-Spio-pan, or Nanomag-D-Spio-pan-IR700) was administered into the tail vein at 80 µL/body (30 µg/body in terms of antibody) (Day-1 in Fig. 12A). From the next day (Day 0 in Fig. 12A), the affected area near the base of the left foot was irradiated with laser light once a day for a total of five times (690 nm, 200 mJ/cm²/time). The size of the tumor after irradiation was evaluated by luciferase assay (BLI) at the timing indicated by "BLI" (arrow in the figure) in Fig. 12A. Fig. 12B shows images of the xenograft model mice observed by the luciferase assay. Fig. 12C is a graph showing a change in tumor size evaluated from the luciferase assay images (n = 6), in which the change in tumor size is expressed as a RLU ratio with the RLU in the region of interest of the tumor image on Day 0 set to 100. Fig. 12D is a graph showing a change in tumor volume measured after tumor resection, and shows a relative value with the tumor size on Day 0 as 100 (n = 6). The tumor volume was significantly reduced at the laser-irradiated site in Nanomag-D-Spio-pan-IR700-administered mice (Dunnet's test with Pan-IR700+NIR-light as a comparative group, p < 0.05).

### Experiment 12: Tumor imaging by antibody-nanoparticle complex

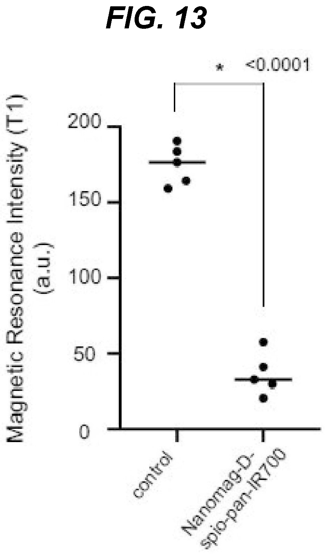

In the same procedure as in Experiment 10, A431-transplanted xenograft model mice were tail intravenously administered with Nanomag-D-Spio-pan-IR700, and the tumors after administration were imaged by MRI. As Control, a mouse to which PBS was tail intravenously administered instead of Nanomag-D-Spio-pan-IR700. The nuclear magnetic resonance intensity (magnetic resonance intensity) of the acquired MRI image is shown in Fig. 13.

### Experiment 13: Production of antibody-nanoparticle complex (Nanomag-D-Spio-Tra)

An antibody-nanoparticle complex (Nanomag-D-Spio-Tra) was produced by the same procedure as in 3) of Experiment 1 using the biotinylated human-type monoclonal antibody trastuzumab (Tra-Biotin) obtained by the same procedure as in 2) of Experiment 1 instead of Pan-IR700-Biotin.

### Experiment 14: Reaction of nanoparticle with methylene blue

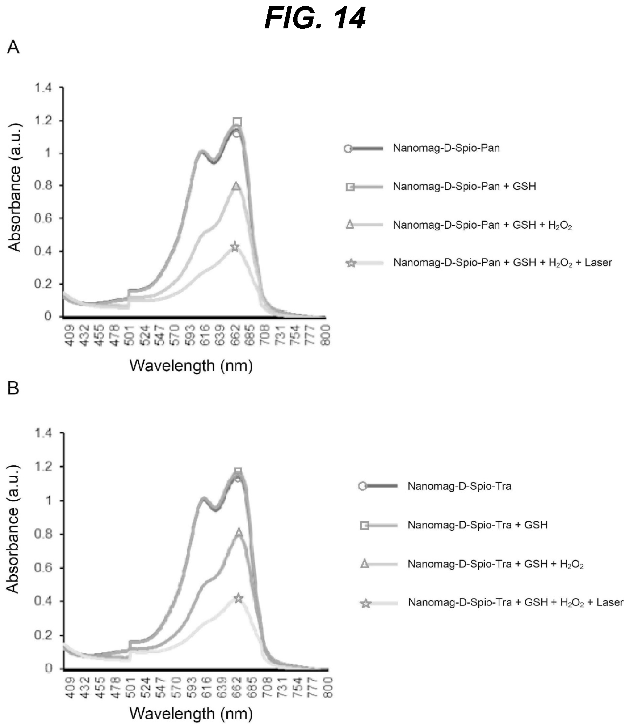

In a microtube, Nanomag-D-Spio-Pan (0.02 mg/mL) or Nanomag-D-Spio-Tra (0.02 mg/mL) was added and heated at 37°C for 30 minutes in the presence or absence of reduced glutathione (GSH, 10 mM). Methylene blue (10 µg/mL) was added to the heated tube with or without hydrogen peroxide (1 mM). The solution in the microtube was adjusted to pH 5.4 with PBS containing HCl and the absorbance (500 to 800 nm) was measured. The solution was irradiated with laser light (690 nm, 10 J/cm², 3 hours), and the absorbance (500 to 800 nm) was measured. The measurement results of absorbance are shown in Fig. 14(A) (Nanomag-D-Spio-Pan) and Fig. 14(B) (Nanomag-D-Spio-Tra). Table 1 shows the absorbance of the nanoparticle complex measured in Experiment 6 and Experiment 14 at a wavelength of 662 nm.

**[Table 1]**

| | Nanomag-D-Spio (Ex. 6) | Nanomag-D-Spio-Pan | Nanomag-D-Spio-Tra |
|---|---|---|---|
| | 0.5 mg/mL | 0.02 mg/mL | 0.02 mg/mL |
| +GSH | 1.22 | 0.42 | 0.42 |
| +H₂O₂ | 1.26 | N/A | N/A |
| +GSH, +H₂O₂ | 1.21 | 0.80 | 0.80 |
| +GSH, +H₂O₂, +Laser (10 J/cm² × 3 hr) | N/A | 1.16 | 1.16 |
| +GSH, +H₂O₂, Laser (200 J/cm² × 2 hr) | 0.59 | N/A | N/A |

## Claims

1. A complex for use in chemodynamic therapy of a tumor, the complex comprising:
a nanoparticle that encloses iron oxide and has a hydrophilic polymer in a surface layer thereof; and
target recognition molecule(s) bonded to the nanoparticle, and is capable of binding to a target molecule of a tumor cell.

2. The complex according to claim 1, wherein the chemodynamic therapy is based on a photo-Fenton reaction generated by near-infrared light irradiation.

3. The complex according to claim 1, wherein the complex is further used for photothermal therapy.

4. The complex according to claim 1, wherein the complex is further used for tumor imaging.

5. The complex according to claim 1, further comprising photosensitive site(s) bonded to the nanoparticle or the target recognition molecule, wherein the photosensitive site contains a photosensitive group having a maximum absorption wavelength at 500 to 1500 nm, and one or more hydrophilic functional groups linked to or coordinated with the photosensitive group.

6. The complex according to claim 5, wherein the photosensitive site is bonded to the target recognition molecule.

7. The complex according to claim 5, which is further used for photoimmunotherapy.

8. The complex according to claim 1, wherein the target recognition molecule is an antibody.

9. The complex according to claim 1, wherein the hydrophilic polymer is a polysaccharide.

10. The complex according to claim 9, wherein the polysaccharide is dextran.

11. The complex according to claim 1, wherein a content of iron atoms in the complex is 30 mass% or more.

12. The complex according to claim 1, wherein the number of bonds of the target recognition molecule to the nanoparticle is 1 to 20 per nanoparticle.

13. The complex according to claim 1, wherein the target molecule of the tumor cell is an epidermal growth factor receptor, an EGF receptor family, or a platelet-activating receptor.

14. A composition for use in chemodynamic therapy of a tumor, the composition comprising the complex according to any one of claims 1 to 13.

15. The composition according to claim 14, further comprising hydrogen peroxide.

16. The composition according to claim 14, further comprising a biological reducing substance.

17. The composition according to claim 14, which is further used for photothermal therapy.

18. The composition according to claim 14, which is further used for tumor imaging.

19. The composition according to claim 14, wherein the complex has photosensitive site(s), and the photosensitive site contains a photosensitive group having a maximum absorption wavelength at 500 to 1500 nm, and one or more hydrophilic functional groups linked to or coordinated with the photosensitive group.

20. The composition according to claim 19, which is further used for photoimmunotherapy.
